(19) **Europäisches Patentamt**
**European Patent Office**
**Office européen des brevets**

(11) **EP 1 644 030 B1**

(12) **EUROPEAN PATENT SPECIFICATION**

(45) Date of publication and mention
of the grant of the patent:
**28.10.2009 Bulletin 2009/44**

(51) Int Cl.:
*A61K 38/48* (2006.01)     *A61K 47/02* (2006.01)
*A61K 47/26* (2006.01)

(21) Application number: **04738962.2**

(86) International application number:
**PCT/DK2004/000465**

(22) Date of filing: **30.06.2004**

(87) International publication number:
**WO 2005/002615 (13.01.2005 Gazette 2005/02)**

(54) **LIQUID, AQUEOUS PHARMACEUTICAL COMPOSITION OF FACTOR VII POLYPEPTIDES**

FLÜSSIGE WÄSSRIGE PHARMAZEUTISCHE ZUSAMMNESETZUNG VON FACTOR VII
POLYPEPTIDEN

PREPARATIONS PHARMACEUTIQUES AQUEUSES LIQUIDES DE POLYPEPTIDES FACTEUR VII

(84) Designated Contracting States:
**AT BE BG CH CY CZ DE DK EE ES FI FR GB GR
HU IE IT LI LU MC NL PL PT RO SE SI SK TR**

(30) Priority: **01.07.2003 DK 200300995
07.07.2003 US 485334 P
18.03.2004 PCT/DK2004/000181**

(43) Date of publication of application:
**12.04.2006 Bulletin 2006/15**

(73) Proprietor: **Novo Nordisk Health Care AG
8050 Zürich (CH)**

(72) Inventors:
• **JENSEN, Michael, Bech
DK-3450 Allerod (DK)**

• **HANSEN, Birthe, Lykkegaard
DK-3500 Vaerlose (DK)**
• **KORNFELT, Troels
DK-2830 Virum (DK)**
• **JAKOBSEN, Kirsten, Kramer
DK-3520 Farum (DK)**

(74) Representative: **Thorsen, Jesper
Inspicos A/S
Kogle Allé 2
P.O. Box 45
2970 Hørsholm (DK)**

(56) References cited:
**EP-A- 0 052 874         EP-A- 0 872 487
WO-A-01/03726         WO-A-94/22905
US-A1- 2002 115 590**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD OF THE INVENTION

The present invention is directed to liquid, aqueous pharmaceutical compositions containing Factor VII polypeptides, and methods for preparing and using such compositions, as well as containers containing such compositions, and the use of such compositions in the treatment of a Factor VII-responsive syndrome. More particularly, the invention relates to liquid compositions stabilised against chemical and/or physical degradation.

BACKGROUND OF THE INVENTION

A variety of Factors involved in the blood clotting process have been identified, including Factor VII (FVII), a plasma glycoprotein. Haemostasis is initiated by the formation of a complex between Tissue Factor (TF) being exposed to the circulating blood following an injury to the vessel wall, and FVIIa which is present in the circulation in an amount corresponding to about 1% of the total FVII protein mass. FVII exists in plasma mainly as a single-chain zymogen which is cleaved by FXa into its two-chain, activated form, FVIIa. Recombinant activated Factor VIIa (rFVIIa) has been developed as a pro-haemostatic agent. The administration of rFVIIa offers a rapid and highly effective pro-haemostatic response in haemophilic subjects with bleedings, who cannot be treated with other coagulation Factor products due to antibody formation. Also bleeding in subjects with Factor VII deficiency or subjects having a normal coagulation system but experiencing excessive bleeding can be treated successfully with FVIIa.

It is desirable to have administration forms of Factor VIIa suitable for both storage and for delivery. Ideally, the drug product is stored and administered as a liquid. Alternatively, the drug product is lyophilized, i.e. freeze-dried, and then reconstituted by adding a suitable diluent prior to patient use. Ideally, the drug product has sufficient stability to be kept in long-term storage, i.e. more than six months.

The decision to either maintain the finished drug product as a liquid or to freeze-dry it is usually based on the stability of the protein drug in those forms. Protein stability can be affected inter alia by such factors as ionic strength, pH, temperature, repeated cycles of freeze/thaw, and exposures to shear forces. Active protein may be lost as a result of physical instabilities, including denaturation and aggregation (both soluble and insoluble aggregate formation), as well as chemical instabilities, including, for example, hydrolysis, deamidation, and oxidation, to name just a few. For a general review of the stability of protein pharmaceuticals, see, for example, Manning, et al., Pharmaceutical Research 6:903-918 (1989).

While the possible occurrence of protein instabilities is widely appreciated, it is impossible to predict particular instability problems of a particular protein. Any of these instabilities can result in the formation of a protein by-product, or derivative, having lowered activity, increased toxicity, and/or increased immunogenicity. Indeed, protein precipitation may lead to thrombosis, non-homogeneity of dosage form and amount, as well as clogged syringes. Furthermore, post-translational modifications such as, for example, gamma-carboxylation of certain glutamic acid residues in the N-terminus and addition of carbohydrate side chains provide potential sites that may be susceptible to modification upon storage. Also, specific to Factor VIIa, being a serine protease, fragmentation due to autocatalysis may occur (enzymatic degradation). Thus, the safety and efficacy of any composition of a protein is directly related to its stability. Maintaining stability in a liquid form is generally different from maintaining stability in a lyophilized form because of highly increased potential for molecular motion and thereby increased probability of molecular interactions. Maintaining stability in a concentrated form is also different from the above, because of the propensity for aggregate formation at increased protein concentrations.

When developing a liquid composition, many factors are taken into consideration. Short-term, i.e. less than six months, liquid stability generally depends on avoiding gross structural changes, such as denaturation and aggregation. These processes are described in the literature for a number of proteins, and many examples of stabilizing agents exist. It is well known that an agent effective in stabilizing one protein actually acts to destabilize another. Once the protein has been stabilized against gross structural changes, developing a liquid composition for long-term stability (e.g., greater than six months) depends on further stabilizing the protein from types of degradation specific to that protein. More specific types of degradation may include, for, example, disulfide bond scrambling, oxidation of certain residues, deamidation, cyclization. Although it is not always possible to pinpoint the individual degradation species, assays are developed to monitor subtle changes so as to monitor the ability of specific excipients to uniquely stabilize the protein of interest.

It is desirable that the pH of the composition is in a physiologically suitable range upon injection/infusion, otherwise pain and discomfort for the patient may result.

For a general review of protein compositions, see, for example, Cleland et al.: The development of stable protein compositions: A closer look at protein aggregation, deamidation and oxidation, Critical Reviews in Therapeutic Drug Carrier Systems 1993, 10(4): 307-377; and Wang et al., Parenteral compositions of proteins and peptides: Stability and stabilizers, Journal of Parenteral Science and Technology 1988 (Supplement), 42 (2S).

Factor VIIa undergoes several degradative pathways, especially aggregation (dimerisation), oxidation, and autolytic cleavage (clipping of the peptide backbone or "heavy chain degradation"). Furthermore, precipitation may occur. Many of these reactions can be slowed significantly by removal of water from the protein. However, the development of an aqueous composition for Factor VIIa has the advantages of eliminating reconstitution errors, thereby Increasing dosing accuracy, as well as simplifying the use of the product clinically, thereby increasing patient compliance. Ideally, compositions of Factor VIIa should be stable for more than 6 months over a wide range of protein concentrations. This allows for flexibility In methods of administration. Generally, more highly concentrated forms allow for the administration of lower volumes, which is highly desirable from the patients' point of view. Liquid compositions can have many advantages over freeze-dried products with regard to ease of administration and use.

Today, the only commercially available, recombinantly-made FVII polypeptide composition is a freeze-dried Factor FVIIa product which is reconstituted before use; it contains a relatively low Factor VIIa concentration, e.g., about 0.6 mg/mL. A vial (1.2 mg) of NovoSeven® (Novo Nordisk A/S, Denmark) contains 1.2 mg recombinant human Factor VIIa, 5.84 mg NaCl, 2.94 mg $CaCl_2$, 2 $H_2O$, 2.64 mg glycylglycine (GlyGly), 0.14 mg polysorbate 80, and 60.0 mg mannitol; it is reconstituted to pH 5.5 by 2.0 mL water for injection (WFI). When reconstituted, the protein solution is stable for use for 24 hours. Thus, no liquid ready-for-use- or concentrated Factor VII products are currently commercially available.

US 2004/115590 A discloses liquid, aqueous compositions of Factor VIIa. It is suggested that the formulation is stabilised with calcium, or other divalent metal ions, e.g. zinc in order to maintain the Factor VIIa activity.

WO 94/22905 A discloses the purification of Factor VII involving a number of chromatographic purification steps, wherein $Zn^{++}$ is present in at least one of the purification steps.

Accordingly, it is an objective of this invention to provide a liquid, aqueous Factor VII polypeptide pharmaceutical composition which provides acceptable control of chemical and/or physical degradation products such as enzymatic degradation or autocatalysis products.

## SUMMARY OF THE INVENTION

The present inventors have discovered that Factor VII or analogues thereof ("Factor VII polypeptides"), when formulated as liquid, aqueous pharmaceutical compositions together with at least one metal-containing agent wherein said metal is selected from the group consisting of chromium, manganese, iron, cobalt, nickel, and copper of oxidation state +II exhibit improved stability and thereby allow for prolonged storage before actual use.

Thus, one aspect of the present invention relates to a liquid, aqueous pharmaceutical composition comprising
a Factor VII polypeptide (i);
a buffering agent (ii) suitable for keeping pH in the range of from about 4.0 to about 9.0;
at least one metal-containing agent (iii), wherein said metal is selected from the group consisting of chromium, manganese, iron, cobalt, nickel, and copper of oxidation state +II; and
a non-ionic surfactant (iv).

A second aspect of the present invention relates to a method for preparing a liquid, aqueous pharmaceutical composition of a Factor VII polypeptide, comprising the step of providing the Factor VII polypeptide (i) in a solution comprising
a buffering agent (ii) suitable for keeping pH in the range of from about 4.0 to about 9.0;
at least one metal-containing agent (iii), wherein said metal is selected from the group consisting of chromium, manganese, iron, cobalt, nickel, and copper of oxidation state +II; and
a non-ionic surfactant (iv).

A third aspect of the present invention relates to the liquid, aqueous pharmaceutical composition for use as a medicament.

A fourth aspect of the present invention relates to the use of the liquid, aqueous pharmaceutical composition for the preparation of a medicament for treating a Factor VII-responsive syndrome.

A fifth aspect of the present invention relates to a method for treating a Factor VII-responsive syndrome, the method comprising administering to a subject in need thereof an effective amount of the liquid, aqueous pharmaceutical composition.

A sixth aspect of the present invention relates to an air-tight container containing the liquid, aqueous pharmaceutical composition and optionally an inert gas

A seventh aspect of the present invention relates to a method of lowering the metal ion concentration in a liquid, aqueous pharmaceutical composition, said method comprising the step of contacting the liquid, aqueous pharmaceutical composition with a cation-exchange material.

DETAILED DESCRIPTION OF THE INVENTION

As mentioned above, the present invention resides in the development of a novel stabilised liquid, aqueous pharmaceutical composition comprising a Factor VII polypeptide. More specifically, the liquid, aqueous pharmaceutical composition comprises

a Factor VII polypeptide (i);

a buffering agent (ii) suitable for keeping pH in the range of from about 4.0 to about 9.0;

at least one metal-containing agent (iii), wherein said metal is selected from the group - consisting of chromium, manganese, iron, cobalt, nickel, and copper of oxidation state +II; and

a non-ionic surfactant (iv).

The present inventors are under the impression that first transition series metals of oxidation state +II, except zinc, have not previously been utilised as stabilising agents in connection with ready-to-use pharmaceutical compositions.

When used herein, the term "first transition series metals of oxidation state +II, except zinc" is intended to encompass the metals titanium, vanadium, chromium, manganese, iron, cobalt, nickel and copper. As described in various text books (e.g. in "Basic Inorganic Chemistry", 2nd ed., Cotton, A. et al., John Wiley & Sons, New York, 1987, Chapter 2-5, page 53), zinc as well as cadmium and mercury have properties different from the remaining metals of the transition metal series. For this reason, zinc is not considered useful in connection with the present invention.

The metal(s) are selected among chromium, manganese, iron, cobalt, nickel, and copper. Illustrative examples of metal-containing agents (iii) corresponding to these metals are chromium(II) chloride, manganese(II) chloride, iron(II) chloride, cobalt(II) chloride, nickel(II) chloride, and copper(II) chloride. It should be understood that the metal-containing agent (iii) may comprise two or more metals, e.g. two or more first transition series metals selected from chromium, manganese, iron, cobalt nickel, and copper. Thus in some instances, two or more of the above-mentioned agents may be used in combination.

So far, the most promising metals are copper and manganese. Illustrative examples of corresponding metal-containing agents (iii) are copper(II) chloride and manganese(II) chloride.

The concentration of the metal-containing agent (or agents) (iii) is typically at least 1 $\mu$M. The desirable (or necessary) concentration typically depends on the selected metal-containing agent (or agents), more specifically on the binding affinity of the selected metal of oxidation state +II to the Factor VII polypeptide.

In different embodiments, the metal-containing agent (iii) is present in a concentration of at least 5 $\mu$M, at least 25 $\mu$M, at least 50 $\mu$M, at least 100 $\mu$M, at least 200 $\mu$M, at last 400 $\mu$M, at least 500 $\mu$M, at least 800 $\mu$M, at least 900 $\mu$M, at least 1000 $\mu$M, at least 5 mM, at least 25 mM, at least 50 mM, at least 100 mM, at least 200 mM, at least 400 mM, at least 800 mM, at least 900 mM, or at least 1000 mM.

In one particular embodiment, the metal of the metal-containing agent (iii) is copper and the concentration of said agent is at least 5 $\mu$M, such as at least 10 $\mu$M, at least 15 $\mu$M, at least 25 $\mu$M, or at least 50 $\mu$M.

In another particular embodiment, the metal of the metal-containing agent (iii) is manganese and the concentration of said agent is at least 100 $\mu$M, such as at least 500 $\mu$M, at least 1 mM, or at least 5 mM.

In various embodiments, the molar ratio between the metal-containing agent (iii) (Me2+) and FVII polypeptide is: above 0.5; above 1; above 2; above 4; above 5; above 10; above 25; above 100; above 150; such as, e.g., in the range of 0.5-250, such as 0.5-150, 0.5-100; 0.5-25; 1-250; 1-100; 1-25; 1-10.

In one embodiment, the composition further contains calcium ($Ca^{2+}$) and/or magnesium ($Mg^{2+}$), such as, for example, selected from a list of: calcium chloride, calcium acetate, calcium gluconate, calcium laevulate, magnesium chloride, magnesium acetate, magnesium sulphate, magnesium gluconate, magnesium laevulate, magnesium salts of strong acids, or mixtures thereof.

In one embodiment, the Calcium ($Ca^{2+}$) and/or Magnesium ($Mg^{2+}$) is present in a concentration of at least about 0.1 $\mu$M, such as, e.g., at least about 0.5 $\mu$M, at least about 1 $\mu$M, at least about 5 $\mu$M, at least about 10 $\mu$M, at least about 50 $\mu$M, at least about 100 $\mu$M, at least about 1 mM, at least about 2 mM, at least about 5 mM, or at least about 10mM. In a particular embodiment the composition comprises at least 2 mM $Ca^{2+}$.

In various embodiments, the molar ratio between calcium (Ca2+) and/or magnesium ions (Mg2+) and FVII polypeptide is: 0.001-750; 0.001-250; 0.001-100; 0.001-10; 0.001-1.0; 0.001-0.5; 0.5-750; 0.5-250; 0.5-100; 0.5-10; 0.5-1.0; 0.001-0.4999; 0.005-0.050.

In one embodiment of the present invention, the molar ratio of non-complexed calcium (Ca2+) and/or magnesium ($Mg^{2+}$) to the Factor VII polypeptide is lower than 0.5, e.g. in the range of 0.001-0.499, such as 0.005-0.050, or in the range of 0.000-0.499, such as in the range of 0.000-0.050, or about 0.000. In one embodiment of the present invention, the molar ratio of non-complexed calcium ($Ca^{2+}$) to the Factor VII polypeptide, is lower than 0.5, e.g. in the range of 0.001-0.499, such as 0.005-0.050, or in the range of 0.000-0.499, such as in the range of 0.000-0.050, or about 0.000.

In another embodiment, the molar ratio of non-complexed calcium and/or magnesium ions to the Factor VII polypeptide is above 0.5. In another embodiment, the molar ratio of non-complexed calcium ions to the Factor VII polypeptide is above 0.5.

In order to obtain the low relative ratio between calcium and/or magnesium ions (Ca2+) and the Factor VII polypeptide, it may be necessary or desirable to remove excess calcium and/or magnesium ions, e.g., by contacting the composition with an ion-exchange material under conditions suitable for removing Ca2+ and/or Mg2+ without removing metal-containing agents (iii). This is particularly relevant where the ratio between calcium and/or magnesium ions and the Factor VII polypeptide in a solution from a process step preceding the formulation step exceeds the limit stated above.

The biological effect of the pharmaceutical composition is mainly ascribed to the presence of the Factor VII polypeptide.

As used herein, the term "Factor VII polypeptide" encompasses wild-type Factor VII (i.e. a polypeptide having the amino acid sequence disclosed in U.S. Patent No. 4,784,950), as well as variants of Factor VII exhibiting substantially the same or improved biological activity relative to wild-type Factor VII. The term "Factor VII" is intended to encompass Factor VII polypeptides in their uncleaved (zymogen) form, as well as those that have been proteolytically processed to yield their respective bioactive forms, which may be designated Factor VIIa. Typically, Factor VII is cleaved between residues 152 and 153 to yield Factor VIIa. The term "Factor VII polypeptide" also encompasses polypeptides, including variants, in which the Factor VIIa biological activity has been substantially modified or somewhat reduced relative to the activity of wild-type Factor VIIa. These polypeptides include, without limitation, Factor VII or Factor VIIa into which specific amino acid sequence alterations have been introduced that modify or disrupt the bioactivity of the polypeptide.

The biological activity of Factor VIIa in blood clotting derives from its ability to (i) bind to Tissue Factor (TF) and (ii) catalyze the proteolytic cleavage of Factor IX or Factor X to produce activated Factor IX or X (Factor IXa or Xa, respectively).

For the purposes of the invention, biological activity of Factor VII polypeptides ("Factor VII biological activity") may be quantified by measuring the ability of a preparation to promote blood clotting using Factor VII-deficient plasma and thromboplastin, as described, e.g., in U.S. Patent No. 5,997,864 or WO 92/15686, or as described in Assay 4 of the present specification (see below). Alternatively, Factor VIIa biological activity may be quantified by (i) measuring the ability of Factor VIIa or a Factor VII-related polypeptide to produce activated Factor X (Factor Xa) in a system comprising TF embedded in a lipid membrane and Factor X. (Persson et al., J. Biol. Chem. 272:19919-19924, 1997); (ii) measuring Factor X hydrolysis in an aqueous system ("In Vitro Proteolysis Assay", Assay 2 below); (iii) measuring the physical binding of Factor VIIa or a Factor VII-related polypeptide to TF using an instrument based on surface plasmon resonance (Persson, FEBS Letts. 413:359-363, 1997); (iv) measuring hydrolysis of a synthetic substrate by Factor VIIa and/or a Factor VII-related polypeptide ("In Vitro Hydrolysis Assay", Assay 1 below); or (v) measuring generation of thrombin in a TF-independent in vitro system (Assay 3 below).

Factor VII variants having substantially the same or improved biological activity relative to wild-type Factor VIIa encompass those that exhibit at least about 25%, preferably at least about 50%, more preferably at least about 75% and most preferably at least about 90% of the specific activity of Factor VIIa that has been produced in the same cell type, when tested in one or more of a clotting assay, proteolysis assay, or TF binding assay as described above. Factor VII variants having substantially reduced biological activity relative to wild-type Factor VIIa are those that exhibit less than about 25%, such as, e.g., less than about 10%, or less than about 5% of the specific activity of wild-type Factor VIIa that has been produced in the same cell type when tested in one or more of a clotting assay, proteolysis assay, or TF binding assay as described above. Factor VII variants having a substantially modified biological activity relative to wild-type Factor VII include, without limitation, Factor VII variants that exhibit TF-independent Factor X proteolytic activity and those that bind TF but do not cleave Factor X.

Variants of Factor VII, whether exhibiting substantially the same or better bioactivity than wild-type Factor VII, or, alternatively, exhibiting substantially modified or reduced bioactivity relative to wild-type Factor VII, include, without limitation, polypeptides having an amino acid sequence that differs from the sequence of wild-type Factor VII by insertion, deletion, or substitution of one or more amino acids.

Non-limiting examples of Factor VII variants having substantially the same biological activity as wild-type Factor VII include S52A-FVIIa, S60A-FVIIa (Lino et al., Arch. Biochem. Biophys. 352: 182-192, 1998); FVIIa variants exhibiting increased proteolytic stability as disclosed in U.S. Patent No. 5,580,560; Factor VIIa that has been proteolytically cleaved between residues 290 and 291 or between residues 315 and 316 (Mollerup et al., Biotechnol. Bioeng. 48:501-505, 1995); oxidized forms of Factor VIIa (Kornfelt et al., Arch. Biochem. Biophys. 363:43-54, 1999); FVII variants as disclosed in PCT/DK02/00189; and FVII variants exhibiting increased proteolytic stability as disclosed in WO 02/38162 (Scripps Research Institute); FVII variants having a modified Gla-domain and exhibiting an enhanced membrane binding as disclosed in WO 99/20767 (University of Minnesota); and FVII variants as disclosed in WO 01/58935 (Maxygen ApS).

Non-limiting examples of Factor VII variants having increased biological activity compared to wild-type FVIIa include FVII variants as disclosed in WO 01/83725, WO 02/22776, WO 02/077218, WO 03/27147, WO 03/37932; WO 02/38162 (Scripps Research Institute); and FVIIa variants with enhanced activity as disclosed in JP 2001061479 (Chemo-Sero-Therapeutic Res Inst.).

Non-limiting examples of Factor VII variants having substantially reduced or modified biological activity relative to wild-type Factor VII include R152E-FVIIa (Wildgoose et al., Biochem 29:3413-3420, 1990).

Explicit examples of Factor VII polypeptides include, without limitation, wild-type Factor VII, L305V-FVII, L305V/M306D/D309S-FVII, L305I-FVII, L305T-FVII, F374P-FVII, V158T/M298Q-FVII, V158D/E296V/M298Q-FVII, K337A-FVII, M298Q-FVII, V158D/M298Q-FVII, L305V/K337A-FVII, V158D/E296V/M298Q/L305V-FVII, V158D/E296V/M298Q/K337A-FVII, V158D/E296V/M298Q/L305V/K337A-FVII, K157A-FVII, E296V-FVII, E296V/M298Q-FVII, V158D/E296V-FVII, V158D/M298K-FVII, and S336G-FVII, L305V/K337A-FVII, L305V/V158D-FVII, L305V/E296V-FVII, L305V/M298Q-FVII, L305V/V158T-FVII, L305V/K337A/V158T-FVII, L305V/K337A/M298Q-FVII, L305V/K337A/E296V-FVII, L305V/K337A/V158D-FVII, L305V/V158D/M298Q-FVII, L305V/V158D/E296V-FVII, L305V/V158T/M298Q-FVII, L305V/V158T/E296V-FVII, L305V/E296V/M298Q-FVII, L305V/V158D/E296V/M298Q-FVII, L305V/V158T/E296V/M298Q-FVII, L305V/V158T/K337A/M298Q-FVII, L305V/V158T/E296V/K337A-FVII, L305V/V158D/K337A/M298Q-FVII, L305V/V158D/E296V/K337A-FVII, L305V/V158D/E296V/M298Q/K337A-FVII, L305V/V158T/E296V/M298Q/K337A-FVII, S314E/K316H-FVII, S314E/K316Q-FVII, S314E/L305V-FVII, S314E/K337A-FVII, S314E/V158D-FVII, S314E/E296V-FVII, S314E/M298Q-FVII, S314E/V158T-FVII, K316H/L305V-FVII, K316H/K337A-FVII, K316H/V158D-FVII, K316H/E296V-FVII, K316H/M298Q-FVII, K316H/V158T-FVII, K316Q/L305V-FVII, K316Q/K337A-FVII, K316Q/V158D-FVII, K316Q/E296V-FVII, K316Q/M298Q-FVII, K316Q/V158T-FVII, S314E/L305V/K337A-FVII, S314E/L305V/V158D-FVII, S314E/L305V/E296V-FVII, S314E/L305V/M298Q-FVII, S314E/L305V/V158T-FVII, S314E/L305V/K337A/V158T-FVII, S314E/L305V/K337A/M298Q-FVII, S314E/L305V/K337A/E296V-FVII, S314E/L305V/K337A/V158D-FVII, S314E/L305V/V158D/M298Q-FVII, S314E/L305V/V158D/E296V-FVII, S314E/L305V/V158T/M298Q-FVII, S314E/L305V/V158T/E296V-FVII, S314E/L305V/E296V/M298Q-FVII, S314E/L305V/V158D/E296V/M298Q-FVII, S314E/L305V/V158T/E296V/M298Q-FVII, 5314E/L305V/V158T/K337A/M298Q-FVII, S314E/L305V/V158T/E296V/K337A-FVII, S314E/L305V/V158D/K337A/M298Q-FVII, S314E/L305V/V158D/E296V/K337A-FVII, S314E/L305V/V158D/E296V/M298Q/K337A-FVII, S314E/L305V/V158T/E296V/M298Q/K337A-FVII, K316H/L305V/K337A-FVII, K316H/L305V/V158D-FVII, K316H/L305V/E296V-FVII, K316H/L305V/M298Q-FVII, K316H/L305V/V158T-FVII, K316H/L305V/K337A/V158T-FVII, K316H/L305V/K337A/M298Q-FVII, K316H/L305V/K337A/E296V-FVII, K316H/L305V/K337A/V158D-FVII, K316H/L305V/V158D/M298Q-FVII, K316H/L305V/V158D/E296V-FVII, K316H/L305V/V158T/M298Q-FVII, K316H/L305V/V158T/E296V-FVII, K316H/L305V/E296V/M298Q-FVII, K316H/L305V/V158D/E296V/M298Q-FVII, K316H/L305V/V158T/E296V/M298Q-FVII, K316H/L305V/V158T/K337A/M298Q-FVII, K316H/L305V/V158T/E296V/K337A-FVII, K316H/L305V/V158D/K337A/M298Q-FVII, K316H/L305V/V158D/E296V/K337A-FVII, K316H/L305V/V158D/E296V/M298Q/K337A-FVII, K316H/L305V/V158T/E296V/M298Q/K337A-FVII, K316Q/L305V/K337A-FVII, K316Q/L305V/V158D-FVII, K316Q/L305V/E296V-FVII, K316Q/L305V/M298Q-FVII, K316Q/L305V/V158T-FVII, K316Q/L305V/K337A/V158T-FVII, K316Q/L305V/K337A/M298Q-FVII, K316Q/L305V/K337A/E296V-FVII, K316Q/L305V/K337A/V158D-FVII, K316Q/L305V/V158D/M298Q-FVII, K316Q/L305V/V158D/E296V-FVII, K316Q/L305V/V158T/M298Q-FVII, K316Q/L305V/V158T/E296V-FVII, K316Q/L305V/E296V/M298Q-FVII, K316Q/L305V/V158D/E296V/M298Q-FVII, K316Q/L305V/V158T/E296V/M298Q-FVII, K316Q/L305V/V158T/K337A/M298Q-FVII, K316Q/L305V/V158T/E296V/K337A-FVII, K316Q/L305V/V158D/K337A/M298Q-FVII, K316Q/L305V/V158D/E296V/K337A -FVII, K316Q/L305V/V158D/E296V/M298Q/K337A-FVII, K316Q/L305V/V158T/E296V/M298Q/K337A-FVII, F374Y/K337A-FVII, F374Y/V158D-FVII, F374Y/E296V-FVII, F374Y/M298Q-FVII, F374Y/V158T-FVII, F374Y/S314E-FVII, F374Y/L305V-FVII, F374Y/L305V/K337A-FVII, F374Y/L305V/V158D-FVII, F374Y/L305V/E296V-FVII, F374Y/L305V/M298Q-FVII, F374Y/L305V/V158T-FVII, F374Y/L305V/S314E-FVII, F374Y/K337A/S314E-FVII, F374Y/K337A/V158T-FVII, F374Y/K337A/M298Q-FVII, F374Y/K337A/E296V-FVII, F374Y/K337A/V158D-FVII, F374Y/V158D/S314E-FVII, F374Y/V158D/M298Q-FVII, F374Y/V158D/E296V-FVII, F374Y/V158T/S314E-FVII, F374Y/V158T/M298Q-FVII, F374Y/V158T/E296V-FVII, F374Y/E296V/S314E-FVII, F374Y/S314E/M298Q-FVII, F374Y/E296V/M298Q-FVII, F374Y/L305V/K337A/V158D-FVII, F374Y/L305V/K337A/E296V-FVII, F374Y/L305V/K337A/M298Q-FVII, F374Y/L305V/K337A/V158T-FVII, F374Y/L305V/K337A/S314E-FVII, F374Y/L305V/V158D/E296V-FVII, F374Y/L305V/V158D/M298Q-FVII, F374Y/L305V/V158D/S314E-FVII, F374Y/L305V/E296V/M298Q-FVII, F374Y/L305V/E296V/V158T-FVII, F374Y/L305V/E296V/S314E-FVII, F374Y/L305V/M298Q/V158T-FVII, F374Y/L305V/M298Q/S314E-FVII, F374Y/L305V/V158T/S314E-FVII, F374Y/K337A/S314E/V158T-FVII, F374Y/K337A/S314E/M298Q-FVII, F374Y/K337A/S314E/E296V-FVII, F374Y/K337A/S314E/V158D-FVII, F374Y/K337A/V158T/M298Q-FVII, F374Y/K337A/V158T/E296V-FVII, F374Y/K337A/M298Q/E296V-FVII, F374Y/K337A/M298Q/V158D-FVII, F374Y/K337A/E296V/V158D-FVII, F374Y/V158D/S314E/M298Q-FVII, F374Y/V158D/S314E/E296V-FVII, F374Y/V158D/M298Q/E296V-FVII, F374Y/V158T/S314E/E296V-FVII, F374Y/V158T/S314E/M298Q-FVII, F374Y/V158T/M298Q/E296V-FVII, F374Y/E296V/S314E/M298Q-FVII, F374Y/L305V/M298Q/K337A/S314E-FVII, F374Y/L305V/E296V/K337A/S314E-FVII, F374Y/E296V/M298Q/K337A/S314E-FVII, F374Y/L305V/E296V/M298Q/K337A -FVII, F374Y/L305V/E296V/M298Q/S314E-FVII, F374Y/V158D/E296V/M298Q/K337A-FVII, F374Y/V158D/E296V/M298Q/S314E-FVII, F374Y/L305V/V158D/K337A/S314E-FVII, F374Y/V158D/M298Q/K337A/S314E-FVII, F374Y/V158D/E296V/K337A/S314E-FVII, F374Y/L305V/V158D/E296V/M298Q-FVII, F374Y/L305V/V158D/M298Q/K337A-FVII, F374Y/L305V/V158D/E296V/K337A-FVII, F374Y/L305V/V158D/M298Q/S314E-FVII, F374Y/L305V/V158D/E296V/S314E-FVII, F374Y/V158T/E296V/M298Q/K337A-FVII, F374Y/V158T/E296V/M298Q/S314E-FVII, F374Y/L305V/V158T/K337A/S314E-FVII, F374Y/V158T/M298Q/K337A/S314E-FVII, F374Y/V158T/E296V/K337A/S314E-FVII,

EP 1 644 030 B1

F374Y/L305V/V158T/E296V/M298Q-FVII, F374Y/L305V/V158T/M298Q/K337A-FVII, F374Y/L305V/V158T/E296V/K337A-FVII, F374Y/L305V/V158T/M298Q/S314E-FVII, F374Y/L305V/V158T/E296V/S314E-FVII, F374Y/E296V/M298Q/K337A/V158T/S314E-FVII, F374Y/V158D/E296V/M298Q/K337A/S314E-FVII, F374Y/L305V/V158D/E296V/M298Q/S314E-FVII, F374Y/L305V/E296V/M298Q/V158T/S314E-FVII, F374Y/L305V/E296V/M298Q/K337A/V158T-FVII, F374Y/L305V/E296V/K337A/V158T/S314E-FVII, F374Y/L305V/M298Q/K337A/V158T/S314E-FVII, F374Y/L305V/V158D/E296V/M298Q/K337A-FVII, F374Y/L305V/V158D/E296V/K337A/S314E-FVII, F374Y/L305V/V158D/M298Q/K337A/S314E-FVII, F374Y/L305V/E296V/M298Q/K337A/V158T/S314E-FVII, F374Y/L305V/V158D/E296V/M298Q/K337A/S314E-FVII, S52A-Factor VII, S60A-Factor VII; R152E-Factor VII, S344A-Factor VII, Factor VIIa lacking the Gla domain; and P11Q/K33E-FVII, T106N-FVII, K143N/N145T-FVII, V253N-FVII, R290N/A292T-FVII, G291N-FVII, R315N/V317T-FVII, K143N/N145T/R315N/V317T-FVII; and FVII having substitutions, additions or deletions in the amino acid sequence from 233Thr to 240Asn, FVII having substitutions, additions or deletions in the amino acid sequence from 304Arg to 329Cys, and FVII having substitutions, deletions, or additions in the amino acid sequence Ile153-Arg223.

In some embodiments, the Factor VII polypeptide is human Factor VIIa (hFVIIa), preferably recombinantly made human Factor VIIa (rhVIIa).

In other embodiments, the Factor VII polypeptide is a Factor VII sequence variant.

In some embodiments, the Factor VII polypeptide has a glycosylation different from wild-type human Factor VII.

In various embodiments, e.g. those where the Factor VII polypeptide is a Factor VII-related polypeptide or a Factor VII sequence variant, the ratio between the activity of the Factor VII polypeptide and the activity of native human Factor VIIa (wild-type FVIIa) is at least about 1.25, preferably at least about 2.0, or 4.0, most preferred at least about 8.0, when tested in the "In Vitro Proteolysis Assay" as described in the present specification.

In some embodiments, the Factor VII polypeptides are Factor VII-related polypeptides, in particular variants, wherein the ratio between the activity of said Factor VII polypeptide and the activity of native human Factor VIIa (wild-type FVIIa) is at least about 1.25 when tested in the "In Vitro Hydrolysis Assay" (see "Assays", below); in other embodiments, the ratio is at least about 2.0; in further embodiments, the ratio is at least about 4.0.

In different embodiments, the Factor VII polypeptide is present in a concentration of 0.1-15 mg/mL; 0.1-10.0 mg/mL; 0.5-5.0 mg/mL; 0.6-4.0 mg/mL; 1.0-4.0 mg/mL; 0.1-5 mg/mL; 0.1-4.0 mg/mL; 0.1-2 mg/mL; or 0.1-1.5 mg/mL.

Factor VIIa concentration is conveniently expressed as mg/mL or as IU/mL, with 1 mg usually representing 43000 - 56000 IU or more.

In order to render the liquid, aqueous pharmaceutical composition useful for direct parenteral administration to a mammal such as a human, it is normally required that the pH value of the composition is held within certain limits, such as from about 4.0 to about 9.0. To ensure a suitable pH value under the conditions given, the pharmaceutical composition also comprises a buffering agent (ii) suitable for keeping pH in the range of from about 4.0 to about 9.0.

The term "buffering agent" encompasses those agents or combinations of agents that maintain the solution pH in an acceptable range from about 4.0 to about 9.0. The term further encompasses agents or combination of agents that has a suitable limited ability to bind the stabilizing divalent metal ions (i.e., a limited formation of metal complexes with the first transition series metal of oxidation state +II according to the invention). In one embodiment the buffering agents or combination of agents and the divalent metal ions in the composition show a binding affinity of about 1% or less compared to the binding affinity of the divalent metal ions towards the Factor VII polypeptide.

In one embodiment, the buffering agent (ii) is at least one component selected from the groups consisting of acids and salts of MES, PIPES, ACES, BES, TES, HEPES, TRIS, glycinamide, histidine (e.g. L-histidine), imidazole, glycine, glycylglycine, glutaric acid, citric acid (e.g. sodium or potassium citrate), tartaric acid, malic acid, maleic acid, phosphoric acid (e.g. sodium or potassium phosphate), acetic acid (e.g. ammonium, sodium or calcium acetate), lactic acid, and succinic acid. It should be understood that the buffering agent may comprise a mixture of two or more components, wherein the mixture is able to provide a pH value in the specified range. As examples can be mentioned acetic acid and sodium acetate, acetic acid and histidine, etc.

The concentration of the buffering agent is chosen so as to maintain the preferred pH of the solution. In various embodiments, the concentration of the buffering agent is 1-100 mM; 1-50 mM; 1-25 mM; or 2-20 mM.

In one embodiment, the pH of the composition is kept from about 4.0 to about 9.0; such as from about 5.0 to about 9.0, from about 4.0 to about 8.0, from about 4.0 to about 7.5, from about 4.0 to about 7.0; from about 4.5 to about 7.5; from about 4.5 to about 7.0; from about 5.0 to about 7.5; from about 5.0 and about 7.0; from about 5.0 to about 6.5; from about 5.0 to about 6.0; from about 5.5 to about 7.5; from about 5.5 to about 7.0; from about 5.5 to about 6.5; from about 6.0 to about 7.5; from about 6.5 to about 7.5; or from about 6.0 to about 7.0; from about 6.4 to about 6.6, or about 6.5, from about 5.2 to about 5.7, or about 5.5.

The pharmaceutical composition also includes a non-ionic surfactant. "Surfactants" (also known as "detergents") generally include those agents which protect the protein from air/solution interface induced stresses and solution/surface induced stresses (e.g. resulting in protein aggregation).

Typical types of non-ionic surfactants are polysorbates, poloxamers, polyoxyethylene alkyl ethers, polyethylene/polypropylene block co-polymers, polyethyleneglycol (PEG), polyxyethylene stearates, and polyoxyethylene castor oils.

Illustrative examples of non-ionic surfactants are Tween®, polysorbate 20, polysorbate 80, Brij-35 (polyoxyethylene dodecyl ether), poloxamer 188, poloxamer 407, PEG8000, Pluronic® polyols, polyoxy 23 lauryl ether, Brij-35, Myrj 49, and Cremophor A.

In one embodiment, the non-ionic surfactant is present in an amount of 0.005-2.0% by weight.

In addition to the four mandatory components, the liquid, aqueous pharmaceutical composition may comprise additional components beneficial for the preparation, formulation, stability, or administration of the composition.

Also, the composition may further comprise a tonicity modifying agent (v).

As used herein, the term "tonicity modifying agent" includes agents which contribute to the osmolality of the solution. Tonicity modifying agent (v) includes at least one selected from the group consisting of neutral salts, amino acids, peptides of 2-5 amino acid residues, monosaccharides, disaccharides, polysaccharides, and sugar alcohols. In some embodiments, the composition comprises two or more of such agents in combination.

By "neutral salt" is meant a salt that is neither an acid nor a base when dissolved in an aqueous solution.

In one embodiment, at least one tonicity modifying agent (v) is a neutral salt selected from the groups consisting of sodium salts, potassium salts, calcium salts, and magnesium salts, such as sodium chloride, potassium chloride, calcium chloride, calcium acetate, calcium gluconate, calcium laevulate, magnesium chloride, magnesium acetate, magnesium gluconate, and magnesium laevulate.

In a further embodiment, the tonicity modifying agent (v) includes sodium chloride in combination with at least one selected from the groups consisting of calcium chloride, calcium acetate, magnesium chloride and magnesium acetate.

In a still further embodiment, the tonicity modifying agent (v) is at least one selected from the group consisting of sodium chloride, calcium chloride, sucrose, glucose, and mannitol.

In different embodiments, the tonicity modifying agent (v) is present in a concentration of at least 1 mM, at least 5 mM, at least 10 mM, at least 20 mM, at least 50 mM, at least 100 mM, at least 200 mM, at least 400 mM, at least 800 mM, at least 1000 mM, at least 1200 mM, at least 1500 mM, at least 1800 mM, at least 2000 mM, or at least 2200 mM.

In one series of embodiments, the tonicity modifying agent (v) is present in a concentration of 5-2200 mM, such as 25-2200 mM, 50-2200 mM, 100-2200 mM, 200-2200 mM, 400-2200 mM, 600-2200 mM, 800-2200 mM, 1000-2200 mM, 1200-2200 mM, 1400-2200 mM, 1600-2200 mM, 1800-2200 mM, or 2000-2200 mM; 5-1800 mM, 25-1800 mM, 50-1800 mM, 100-1800 mM, 200-1800 mM, 400-1800 mM, 600-1800 mM, 800-1800 mM, 1000-1800 mM, 1200-1800 mM, 1400-1800 mM, 1600-1800 mM; 5-1500 mM, 25-1400 mM, 50-1500 mM, 100-1500 mM, 200-1500 mM, 400-1500 mM, 600-1500 mM, 800-1500 mM, 1000-1500 mM, 1200-1500 mM; 5-1200 mM, 25-1200 mM, 50-1200 mM, 100-1200 mM, 200-1200 mM, 400-1200 mM, 600-1200 mM, or 800-1200 mM.

In one embodiment of the invention, at least one tonicity modifying agent (v) is an ionic strength modifying agent (v/a).

As used herein, the term "ionic strength modifying agent" includes agents, which contribute to the ionic strength of the solution. The agents include, but are not limited to, neutral salts, amino acids, peptides of 2 to 5 amino acid residues. In some embodiments, the composition comprises two or more of such agents in combination.

Non-limiting examples of ionic strength modifying agents (v/a) are neutral salts such as sodium chloride, potassium chloride, calcium chloride and magnesium chloride. In one embodiment, the agent (v/a) is sodium chloride.

The term "ionic strength" is the ionic strength of the solution ($\mu$) which is defined by the equation: $\mu = \frac{1}{2} \Sigma ([i](Z_i^2))$, where $\mu$ is the ionic strength, $[I]$ is the millimolar concentration of an ion, and $Z_i$ is the charge (+ or -) of that ion (see, for example, Solomon, Journal of Chemical Education, 78(12): 1691-92, 2001; James Fritz and George Schenk: Quantitative Analytical Chemistry, 1979).

In different embodiments of the invention, the ionic strength of the composition is at least 50 mM, such as at least 75 mM, at least 100 mM, at least 150 mM, at least 200 mM, at least 250 mM, at least 400 mM, at least 500 mM, at least 650 mM, at least 800 mM, at least 1000 mM, at least 1200 mM, at least 1600 mM, at least 2000 mM, at least 2400 mM, at least 2800 mM, or at least 3200 mM.

In some specific embodiments, the total concentration of the tonicity modifying agent (v) and the ionic strength modifying agent (v/a) is in the range of 1-1000 mM, such as 1-500 mM, 1-300 mM, 10-200 mM, or 20-150 mM; or such as 100-1000 mM, 200-800 mM, or 500-800 mM, depending on the effect any other ingredients may have on the tonicity and ionic strength.

In one embodiment, the composition is isotonic; in another, it is hypertonic.

The term "isotonic" means "isotonic with serum", i.e. at about 300 ± 50 milliosmol/kg. The tonicity is meant to be a measure of osmolality of the solution prior to administration. The term "hypertonic" is meant to designate levels of osmolality above the physiological level of serum, such as levels above 300 ± 50 milliosmol/kg.

Also, a particular embodiment of the present invention relates to the combination of the metal-containing agent (iii) with a fairly high concentration of an ionic strength modifying agent (v/a) selected from the group consisting of sodium salts, calcium salts and magnesium salts. In this embodiment, the ionic strength modifying agent (v/a), i.e. the sodium salt, calcium salt and/or magnesium salt, is present in a concentration of 15-1500 mM, such as 15-1000 mM, 25-1000 mM, 50-1000 mM, 100-1000 mM, 200-1000 mM, 300-1000 mM, 400-1000 mM, 500-1000 mM, 600-1000 mM, 700-1000 mM; 15-800 mM, 25-800 mM, 50-800 mM, 100-800 mM, 200-800 mM, 300-800 mM, 400-800 mM, 500-800 mM; 15-600 mM, 25-600 mM, 50-600 mM, 100-600 mM, 200-600 mM, 300-600 mM; 15-400 mM, 25-400 mM, 50-400 mM, or 100-400 mM.

Within these embodiments, sodium salt may be sodium chloride, the calcium salt may be selected from the group consisting of calcium chloride, calcium acetate, calcium gluconate, and calcium laevulate, and the magnesium salt may be selected from the group consisting of magnesium chloride, magnesium acetate, magnesium gluconate, magnesium laevulate, and magnesium salts of strong acids. In a more specific embodiment, a calcium salt and/or a magnesium salt is/are used in combination with sodium chloride.

In a further embodiment, the composition further comprises (vi) an antioxidant. In different embodiments, the antioxidant is selected from the group consisting of L-methionine, D-methionine, methionine analogues, methionine-containing peptides, methionine-homologues, ascorbic acid, cysteine, homocysteine, gluthatione, cystine, and cysstathionine. In a preferred embodiment, the antioxidant is L-methionine.

The concentration of the antioxidant is typically 0.1-5.0 mg/mL, such as 0.1-4.0 mg/mL, 0.1-3.0 mg/mL, 0.1-2.0 mg/ml, or 0.5-2.0 mg/mL.

Although the examples of antioxidants above are applicable in the present invention, it is envisaged that a number of the specific compounds, e.g. methionine, may form complexes with the metal ions of the metal-containing agent(s) (iii). This may result in a slightly lower effective concentration of the metal-containing agent(s) (iii).

For this reason, in particular embodiments the composition does not include an antioxidant; instead the susceptibility of the Factor VII polypeptide to oxidation is controlled by exclusion of atmospheric air. The use of an antioxidant may of course also be combined with the controlled exclusion of atmospheric air.

Thus, the present invention also provides an air-tight container (e.g. a vial or a cartridge (such as a cartridge for a pen applicator or syringe assembly)) containing a liquid, aqueous pharmaceutical composition as defined herein, and optionally an inert gas.

The inert gas may be selected from the groups consisting of nitrogen, argon, etc. The container (e.g. vial or cartridge) is typically made of glass or plastic, in particular glass, optionally closed by a rubber septum or other closure means allowing for penetration with preservation the integrity of the pharmaceutical composition. In a particular embodiment hereof, the composition does not comprise an antioxidant (vi). In a further embodiment, the container is a vial or cartridge enclosed in a sealed bag, e.g. a sealed plastic bag, such as a laminated (e.g. metal (such as aluminium) laminated plastic bag).

In addition to the mandatory components, the tonicity modifying agent (v) and the optional antioxidant (vi), the pharmaceutical composition may further comprise a preservative (vii).

A preservative may be included in the composition to retard microbial growth and thereby allow "multiple use" packaging of the FVII polypeptides. Examples of preservatives include phenol, benzyl alcohol, orto-cresol, meta-cresol, para-cresol, methyl paraben, propyl paraben, benzalkonium chloride, and benzethonium chloride. The preservative is normally included at a concentration of 0.1-20 mg/mL depending on the pH range and type of preservative.

Still further, the composition may also include one or more agents capable of inhibiting deamidation and isomerisation.

In one embodiment, the liquid, aqueous pharmaceutical composition comprises:

0.1-15 mg/mL of a Factor VII polypeptide (i);

a buffering agent (ii) suitable for keeping pH in the range of from about 4.0 to about 9.0;

a copper-containing agent (iii) in concentration of at least 5 $\mu$M;

a non-ionic surfactant (iv); and

a tonicity modifying agent (v) in a concentration of at least 5 mM.

In another embodiment, the liquid, aqueous pharmaceutical composition comprises:

0.1-15 mg/mL of a Factor VII polypeptide (i);

a buffering agent (ii) suitable for keeping pH in the range of from about 4.0 to about 9.0;

a manganese-containing agent (iii) in concentration of at least 100 $\mu$M;

a non-ionic surfactant (iv); and

at least one tonicity modifying agent (v) in a concentration of at least 5 mM.

As used herein, pH values specified as "about" are understood to be $\pm$ 0.1, e.g. about pH 8.0 includes pH 8.0 $\pm$ 0.1.

Percentages are (weight/weight) both when referring to solids dissolved in solution and liquids mixed into solutions. For example, for Tween, it is the weight of 100% stock/weight of solution.

The compositions according to the present invention are useful as stable and preferably ready-to-use compositions of Factor VII polypeptides. Furthermore, it is believed that the principles, guidelines and specific embodiments given herein are equally applicable for bulk storage of Factor VII polypeptides, *mutatis mutandis.* The compositions are typically stable for at least six months, and preferably up to 36 months; when stored at temperatures ranging from 2°C to 8°C. The compositions are chemically and/or physically stable, in particular chemically stable, when stored for at least 6 months at from 2°C to 8°C.

The term stable" is intended to mean that (i) after storage for 6 months at 2°C to 8°C the composition retains at least 50% of its initial biological activity, e.g., as measured by a one-stage clot assay essentially as described in Assay 4 of the present specification; or (ii) after storage for 6 months at 2°C to 8°C the increase in content of heavy chain degradation products is at the most 40% (w/w) of the initial content of Factor VII polypeptide.

The term "initial content" relates to the amount of Factor VII polypeptides added to a composition upon preparation of the composition.

In various embodiments, the stable composition retains at least 70%, such as at least 80%, or at least 85%, or at least 90%, or at least 95%, of its initial biological activity after storage for 6 months at 2 to 8°C.

In various embodiments the increase in content of heavy chain degradation products in stable compositions is not more than about 30% (w/w), not more than about 25% (w/w), not more than about 20% (w/w), not more than about 15% (w/w), not more than about 10% (w/w), not more than about 5% (w/w), or not more than about 3% (w/w) of the initial content of Factor VII polypeptide.

For the purpose of determining the content of heavy chain degradation products, a reverse phase HPLC was run on a proprietary 4.5x250 mm butylbonded silica column with a particle size of 5 $\mu$m and pore size 300Å. Column temperature: 70°C. A-buffer: 0.1% v/v trifluoracetic acid. B-buffer: 0.09% v/v trifluoracetic acid, 80% v/v acetonitrile. The column was eluted with a linear gradient from X to (X+13)% B in 30 minutes. X was adjusted so that FVIIa elutes with a retention time of approximately 26 minutes. Flow rate: 1.0 mL/min. Detection: 214 nm. Load: 25 $\mu$g FVIIa.

The term "physical stability" of Factor VII polypeptides relates to the formation of insoluble and/or soluble aggregates in the form of dimeric, oligomeric and polymeric forms of Factor VII polypeptides as well as any structural deformation and denaturation of the molecule. Physically stable composition encompasses compositions which remains visually clear. Physical stability of the compositions is often evaluated by means of visual inspection and turbidity after storage of the composition at different temperatures for various time periods. Visual inspection of the compositions is performed in a sharp focused light with a dark background. A composition is classified as physically unstable, when it shows visual turbidity.

The term "chemically stable" is intended to encompass a composition which retains at least 50% of its initial biological activity after storage for 6 months at 2 to 8°C, e.g., as measured by a one-stage coagulation assay essentially as described in Assay 4 of the present specification.

The term "chemical stability" is intended to relate to the formation of any chemical change in the Factor VII polypeptides upon storage in solution at accelerated conditions. Examples are hydrolysis, deamidation and oxidation as well as enzymatic degradation resulting in formation of fragments of Factor VII polypeptides. In particular, the sulphur-containing amino acids are prone to oxidation with the formation of the corresponding sulphoxides.

In a further aspect, the invention also provides a method for preparing a liquid, aqueous pharmaceutical composition of a Factor VII polypeptide, comprising the step of providing the Factor VII polypeptide (i) in a solution comprising a buffering agent (ii) suitable for keeping pH in the range of from about 4.0 to about 9.0; at least one metal-containing agent (iii), wherein said metal is selected from the group consisting of chromium, manganese, iron, cobalt, nickel, and copper of oxidation state +II; and a non-ionic surfactant (iv).

*Methods of use*

As will be understood, the liquid, aqueous pharmaceutical compositions defined herein can be used in the field of medicine. Thus, the present invention in particular provides the liquid, aqueous pharmaceutical compositions defined herein for use as a medicament, more particular for use as a medicament for treating a Factor VII-responsive syndrome.

Consequently, the present invention also provides the use of the liquid, aqueous pharmaceutical composition as defined herein for the preparation of a medicament for treating a Factor VII-responsive syndrome, as well as a method for treating a Factor VII-responsive syndrome, the method comprising administering to a subject in need thereof an effective amount of the liquid, aqueous pharmaceutical composition as defined herein.

The preparations of the present invention may be used to treat any Factor VII-responsive syndrome, such as, e.g., bleeding disorders, including those caused by clotting Factor deficiencies (e.g., e.g. haemophilia A, haemophilia B, coagulation Factor XI deficiency, coagulation Factor VII deficiency); by thrombocytopenia or von Willebrand's disease, or by clotting Factor inhibitors, and intra cerebral haemorrhage, or excessive bleeding from any cause. The preparations may also be administered to patients in association with surgery or other trauma or to patients receiving anticoagulant therapy.

The term "effective amount" is the effective dose to be determined by a qualified practitioner, who may titrate dosages to achieve the desired response. Factors for consideration of dose will include potency, bioavailability, desired pharmacokinetic/pharmacodynamic profiles, condition of treatment, patient-related factors (e.g. weight, health, age, etc.), presence of co-administered medications (e.g., anticoagulants), time of administration, or other factors known to a medical practitioner.

The term "treatment" is defined as the management and care of a subject, e.g. a mammal, in particular a human, for the purpose of combating the disease, condition, or disorder and includes the administration of a Factor VII polypeptide to prevent the onset of the symptoms or complications, or alleviating the symptoms or complications, or eliminating the disease, condition, or disorder. Pharmaceutical compositions according to the present invention containing a Factor VII polypeptide may be administered parenterally to subjects in need of such a treatment. Parenteral administration may be performed by subcutaneous, intramuscular or intravenous injection by means of a syringe, optionally a pen-like syringe. Alternatively, parenteral administration can be performed by means of an infusion pump.

In important embodiments, the pharmaceutical composition is adapted to subcutaneous, intramuscular or intravenous injection according to methods known in the art.

The possibly high concentration of salts in the pharmaceutical compositions defined herein may be disadvantageous for certain groups of patients. The present invention therefore also provides a prior-to-use method for lowering the salt concentration in a liquid, aqueous pharmaceutical composition, wherein said method comprises the step of contacting the liquid, aqueous pharmaceutical composition defined herein with an ion-exchange material, a suitable material for desalting, and/or the step of diluting the composition.

The possibly high concentration of metal ions in the pharmaceutical compositions defined herein may be disadvantageous for certain groups of patients. The present invention therefore also provides a prior-to-use method for lowering the metal ion concentration in a liquid, aqueous pharmaceutical composition, wherein said method comprises the step of contacting the liquid, aqueous pharmaceutical composition defined herein with a cation-exchange material.

An example of a cation-exchange material is Chelex-100 (Fluka-Riedel/Sigma-Aldrich). The cation-exchange material, e.g. Chelex-100, is preferably contained in a sterile container, e.g. in a glass or plastic cartridge.

It is envisaged that the liquid, aqueous pharmaceutical composition is contacted with the cation-exchange material, e.g. by passage through a cartridge containing the cation-exchange material, immediately prior to use. In a particular embodiment, it is envisaged that the cartridge is an integral part of a syringe assembly.

EXPERIMENTALS

*General methods*

*Assays suitable for determining biological activity of Factor VII polypeptides*

Factor VII polypeptides useful in accordance with the present invention may be selected by suitable assays that can be performed as simple preliminary in vitro tests. Thus, the present specification discloses a simple test (entitled "In Vitro Hydrolysis Assay") for the activity of Factor VII polypeptides.

*In Vitro Hydrolysis Assay (assay 1)*

Native (wild-type) Factor VIIa and Factor VII polypeptide (both hereinafter referred to as "Factor VIIa") may be assayed for specific activities. They may also be assayed in parallel to directly compare their specific activities. The assay is carried out in a microtiter plate (MaxiSorp, Nunc, Denmark). The chromogenic substrate D-Ile-Pro-Arg-p-nitroanilide (S-2288, Chromogenix, Sweden), final concentration 1 mM, is added to Factor VIIa (final concentration 100 nM) in 50 mM HEPES, pH 7.4, containing 0.1 M NaCl, 5 mM $CaCl_2$ and 1 mg/mL bovine serum albumin. The absorbance at 405 nm is measured continuously in a SpectraMax™ 340 plate reader (Molecular Devices, USA). The absorbance developed during a 20-minute incubation, after subtraction of the absorbance in a blank well containing no enzyme, is used for calculating the ratio between the activities of Factor VII polypeptide and wild-type Factor VIIa:

$$Ratio = (A405\ nm\ Factor\ VII\ polypeptide)/(A405\ nm\ Factor\ VIIa\ wild\text{-}type).$$

Based thereon, Factor VII polypeptides with an activity lower than, comparable to, or higher than native Factor VIIa may be identified, such as, for example, Factor VII polypeptides where the ratio between the activity of the Factor VII polypeptide and the activity of native Factor VII (wild-type FVII) is about 1.0 versus above 1.0.

The activity of the Factor VII polypeptides may also be measured using a physiological substrate such as Factor X ("In Vitro Proteolysis Assay"), suitably at a concentration of 100-1000 nM, where the Factor Xa generated is measured after the addition of a suitable chromogenic substrate (eg. S-2765). In addition, the activity assay may be run at physiological temperature.

*In Vitro Proteolysis Assay (assay 2)*

Native (wild-type) Factor VIIa and Factor VII polypeptide (both hereinafter referred to as "Factor VIIa") are assayed in parallel to directly compare their specific activities. The assay is carried out in a microtiter plate (MaxiSorp, Nunc, Denmark). Factor VIIa (10 nM) and Factor X (0.8 microM) in 100 $\mu$L 50 mM HEPES, pH 7.4, containing 0.1 M NaCl, 5 mM CaCl$_2$ and 1 mg/mL bovine serum albumin, are incubated for 15 min. Factor X cleavage is then stopped by the addition of 50 $\mu$L 50 mM HEPES, pH 7.4, containing 0.1 M NaCl, 20 mM EDTA and 1 mg/mL bovine serum albumin. The amount of Factor Xa generated is measured by addition of the chromogenic substrate Z-D-Arg-Gly-Arg-p-nitroanilide (S-2765, Chromogenix, Sweden), final concentration 0.5 mM. The absorbance at 405 nm is measured continuously in a SpectraMax™ 340 plate reader (Molecular Devices, USA). The absorbance developed during 10 minutes, after subtraction of the absorbance in a blank well containing no FVIIa, is used for calculating the ratio between the proteolytic activities of Factor VII polypeptide and wild-type Factor VIIa:

$$Ratio = (A405\ nm\ Factor\ VII\ polypeptide)/(A405\ nm\ Factor\ VIIa\ wild\text{-}type).$$

Based thereon, Factor VII polypeptide with an activity lower than, comparable to, or higher than native Factor VIIa may be identified, such as, for example, Factor VII polypeptides where the ratio between the activity of the Factor VII polypeptide and the activity of native Factor VII (wild-type FVII) is about 1.0 versus above 1.0.

The ability of Factor VIIa or Factor VII polypeptides to generate thrombin can also be measured in an assay (*Assay 3*) comprising all relevant coagulation Factors and inhibitors at physiological concentrations (minus Factor VIII when mimicking hemophilia A conditions) and activated platelets (as described on p. 543 in Monroe et al. (1997) Brit. J. Haematol. 99, 542-547).

The biological activity of the Factor VII polypeptides may also be measured using a *one-stage coagulation assay (Assay 4).* For this purpose, the sample to be tested is diluted in 50 mM Pipes-buffer (pH 7.5), 0.1% BSA and 40 $\mu$l is incubated with 40 $\mu$l of Factor VII deficient plasma and 80 $\mu$l of human recombinant tissue factor containing 10 mM Ca$^{2+}$ and synthetic phospholipids. Coagulation times are measured and compared to a standard curve using a reference standard in a parallel line assay.

*Preparation and purification of Factor VII polypeptides*

Human purified Factor VIIa suitable for use in the present invention is preferably made by DNA recombinant technology, e.g. as described by Hagen et al., Proc.Natl.Acad.Sci. USA 83: 2412-2416, 1986, or as described in European Patent No. 0 200 421 (ZymoGenetix, Inc:).

Factor VII may also be produced by the methods described by Broze and Majerus, J.Biol.Chem. 255 (4): 1242-1247, 1980 and Hedner and Kisiel, J.Clin.Invest. 71: 1836-1841, 1983. These methods yield Factor VII without detectable amounts of other blood coagulation Factors. An even further purified Factor VII preparation may be obtained by including an additional gel filtration as the final purification step. Factor VII is then converted into activated Factor VIIa by known means, e.g. by several different plasma proteins, such as Factor XIIa, IX a or Xa. Alternatively, as described by Bjoern et al. (Research Disclosure, 269 September 1986, pp. 564-565), Factor VII may be activated by passing it through an ion-exchange chromatography column, such as Mono Q® (Pharmacia fine Chemicals) or the like, or by autoactivation in solution.

Factor VII-related polypeptides may be produced by modification of wild-type Factor VII or by recombinant technology. Factor VII-related polypeptides with altered amino acid sequence when compared to wild-type Factor VII may be produced by modifying the nucleic acid sequence encoding wild-type Factor VII either by altering the amino acid codons or by removal of some of the amino acid codons in the nucleic acid encoding the natural Factor VII by known means, e.g. by site-specific mutagenesis.

It will be apparent to those skilled in the art that substitutions can be made outside the regions critical to the function of the Factor VIIa molecule and still result in an active polypeptide. Amino acid residues essential to the activity of the Factor VII polypeptide, and therefore preferably not subject to substitution, may be identified according to procedures known in the art, such as site-directed mutagenesis or alanine-scanning mutagenesis (see, e.g., Cunningham and Wells, 1989, Science 244: 1081-1085). In the latter technique, mutations are introduced at every positively charged residue in the molecule, and the resultant mutant molecules are tested for coagulant, respectively cross-linking activity to identify amino acid residues that are critical to the activity of the molecule. Sites of substrate-enzyme interaction can also be determined by analysis of the three-dimensional structure as determined by such techniques as nuclear magnetic resonance analysis, crystallography or photoaffinity labelling (see, e.g., de Vos et al., 1992, Science 255: 306-312; Smith et al., 1992, Journal of Molecular Biology 224: 899-904; Wlodaver et al., 1992, FEBS Letters 309: 59-64).

The introduction of a mutation into the nucleic acid sequence to exchange one nucleotide for another nucleotide may be accomplished by site-directed mutagenesis using any of the methods known in the art. Particularly useful is the procedure that utilizes a super coiled, double stranded DNA vector with an insert of interest and two synthetic primers containing the desired mutation. The oligonucleotide primers, each complementary to opposite strands of the vector, extend during temperature cycling by means of Pfu DNA polymerase. On incorporation of the primers, a mutated plasmid containing staggered nicks is generated. Following temperature cycling, the product is treated with DpnI which is specific for methylated and hemi-methylated DNA to digest the parental DNA template and to select for mutation-containing synthesized DNA. Other procedures known in the art for creating, identifying and isolating variants may also be used, such as, for example, gene shuffling or phage display techniques.

Separation of polypeptides from their cell of origin may be achieved by any method known in the art, including, without limitation, removal of cell culture medium containing the desired product from an adherent cell culture; centrifugation or filtration to remove non-adherent cells; and the like.

Optionally, Factor VII polypeptides may be further purified. Purification may be achieved using any method known in the art, including, without limitation, affinity chromatography, such as, e.g., on an anti-Factor VII antibody column (see, e.g., Wakabayashi et al., J. Biol. Chem. 261:11097, 1986; and Thim et al., Biochem. 27:7785, 1988); hydrophobic interaction chromatography; ion-exchange chromatography; size exclusion chromatography; electrophoretic procedures (e.g., preparative isoelectric focusing (IEF), differential solubility (e.g., ammonium sulfate precipitation), or extraction and the like. See, generally, Scopes, Protein Purification, Springer-Verlag, New York, 1982; and Protein Purification, J.C. Janson and Lars Ryden, editors, VCH Publishers, New York, 1989. Following purification, the preparation preferably contains less than 10% by weight, more preferably less than 5% and most preferably less than 1%, of non-Factor VII polypeptides derived from the host cell.

Factor VII polypeptides may be activated by proteolytic cleavage, using Factor XIIa or other proteases having trypsin-like specificity, such as, e.g., Factor IXa, kallikrein, Factor Xa, and thrombin. See, e.g., Osterud et al., Biochem. 11:2853 (1972); Thomas, U.S. Patent No. 4,456,591; and Hedner et al., J. Clin. Invest. 71:1836 (1983). Alternatively, Factor VII polypeptides may be activated by passing it through an ion-exchange chromatography column, such as Mono Q® (Pharmacia) or the like, or by autoactivation in solution. The resulting activated Factor VII polypeptide may then be formulated and administered as described in the present application.

The following examples illustrate practice of the invention.

*Working Examples*

*Example 1*

Effect of addition of copper- containing and manganese-containing agents to aqueous rFVIIa solutions on heavy chain degradation (autocatalytic cleavage)

In order to investigate the effect of metal ions on rFVIIa, the following procedure was followed:

rFVIIa was transferred to the following solutions by desalting on a PD-10 column (Amersham Biosciences):

| | |
|---|---|
| rFVIIa | 1.0 mg/mL |
| Sodium chloride | 2.92 mg/mL (50 mM) |
| Calcium chloride 2 $H_2O$ | 1.47 mg/mL (10 mM) |
| PIPES | 15.12 mg/mL (50 mM) |
| 1 M NaOH | added to pH 6.5 |

Two solutions of metal-containing agents were prepared:

| Copper(II) chloride, 2 H$_2$O | 10 mM |
| Manganese chloride, 2 H$_2$O | 2 M |

The copper-containing and manganese-containing agents, respectively, were added to the desalted rFVIIa solution in order to reach the concentrations outlined in Table 1. pH vas adjusted to 6.5. The formulations were stored at a temperature of 5°C and analyses were performed at the times indicated in Table 1.

Table 1 - Content of Heavy chain degradation products (%) in rFVIIa formulations containing Cu(II) or Mn(II)

| | Storage at 5°C | | | | |
|---|---|---|---|---|---|
| | T = 0 | T = ½ month | T = 1 month | T = 2 months | T = 3 months |
| Reference* | 10.2 | n.a. | 17.0 | 23.8 | 30.4 |
| [Cu(II)] = 20 $\mu$M | 11.6 | 14.6 | 15.7 | 22.2 | n.a. |
| [Cu(II)] = 80 $\mu$M | 11.5 | 12.7 | 13.3 | 16.9 | 18.1 |
| [Mn(II)] = 2 mM | 11.6 | 13.4 | 14.2 | 18.7 | 21.2 |
| [Mn(II)] = 10 mM | 11.5 | 12.9 | 13.6 | 17.4 | 19.8 |
| * The reference contained 1.0 mg/mL rFVIIa, 10 mM histidine, 10 mM sodium acetate, 10 mM glycylglycine, 50 mM sodium chloride, 10 mM calcium chloride, pH 6.5. | | | | | |

As it can be seen from Table 1, the increase in the content of Heavy chain degradation products in the reference formulation totalled 20.2%-points, whereas the increase in the content of Heavy chain degradation products in the illustrative compositions was 6.6%-points ([Cu(II)] = 80 $\mu$M) and 8.3%-points ([Mn(II)] = 10 mM), respectively.

The content of heavy chain degradation products is determined by RP-HPLC as described in the following:

Reverse phase HPLC was run on a proprietary 4.5x250 mm butylbonded silica column with a particle size of 5 $\mu$m and pore size 300Å. Column temperature: 70°C. A-buffer: 0.1% v/v trifluoracetic acid. B-buffer: 0.09% v/v trifluoracetic acid, 80% v/v acetonitrile. The column was eluted with a linear gradient from X to (X+13)% B in 30 minutes. X was adjusted so that FVIIa elutes with a retention time of approximately 26 minutes. Flow rate: 1.0 mL/min. Detection: 214 nm. Load: 25 $\mu$g FVIIa.

*Example 2*

Addition of copper-containing and manganese-containing agents and to aqueous rFVIIa solutions at high ionic strength

In order to investigate the effect of metal ions and high ionic strength on the stability of rFVIIa, the following procedure can be followed:
rFVIIa is transferred to the following solutions by desalting on a PD-10 column (Amersham Biosciences) :

| Sodium chloride | 2.92 mg/mL (50 mM) |
| Calcium chloride 2 H$_2$O | 29.4 mg/mL (200 mM) |
| PIPES | 15.12 mg/mL (50 mM) |
| Poloxamer 188 | 1.0 mg/mL |
| Methionine | 0.5 mg/mL |
| 1 M NaOH/1 M HCl | added to pH 6.5 |

and/or

| Sodium chloride | 29.2 mg/mL (500 mM) |
| Calcium chloride 2 H$_2$O | 1.47 mg/mL (10 mM) |
| PIPES | 15.12 mg/mL (50 mM) |
| Poloxamer 188 | 1.0 mg/mL |
| Methionine | 0.5 mg/mL |

1 M NaOH/1 M HCl          added to pH 6.5

In both solutions the concentration of Factor VIIa (rFVIIa) is 1.0. mg/mL

Two solutions of metal-containing agents are prepared:

Copper(II) chloride, 2$H_2$O      10 mM
Manganese chloride, 2 $H_2$O     2 M

The copper-containing and manganese-containing agents, respectively, are added to the desalted rFVIIa solution in order to reach the concentrations outlined in example 1. pH is adjusted to 6.5. The formulations are filled in a vial and sealed, optionally under an inert gas. In addition, the vial can be put in an airtight bag to prevent atmospheric air from entering the container. The vials are stored at a temperature of 5°C and analyses are performed as described in and at the times indicated in example 1.

It will be seen that addition of metal (copper or manganese) decreases the formation of heavy chain degradation products. By combining metal (copper or manganese) with high ionic strength the lowest increase will be obtained.

_Example 3_

Addition of copper-containing agents to aqueous rFVIIa solutions with high ionic strength.

In order to investigate the possible synergistic effect of metal ions and high ionic strength on the stability of rFVIIa, the following procedure was followed:

rFVIIa was transferred into various solutions (formulations) as listed in table 1 below in a two step process:

First, rFVIIa was transferred into the various solutions without copper added by desalting on a PD-10 column (Amersham Biosciences). Next, the copper content was obtained in solution 2 and 4 by adding a solution of 10 mM Copper(II) chloride, 2H2O until the stated concentration was reached.

After the copper solution had been added, pH was adjusted to 6.5 and the formulations were filled in cartridges.

The cartridges were stored at a temperature of 5°C and analyses were performed after 0, 0.5, 1, 2, and 3 months as indicated in table 2.

Table 1: Formulations

| Formulations | 1 | 2 | 3 | 4 |
|---|---|---|---|---|
| rFVIIa | 1,0 mg/ml | 1,0 mg/ml | 1,0 mg/ml | 1,0 mg/ml |
| CaCl2, 2H2O | 10mM | 10mM | 200mM | 200mM |
| PIPES-di-Na | 50 mM | 50 mM | 50 mM | 50 mM |
| Cu(II) | - | 80$\mu$M | - | 80$\mu$M |
| pH | 6.50 | 6.50 | 6.50 | 6.50 |

Table 2: Heavy chain degradation (%)

| Formulation | Storage time (months) | | | | |
|---|---|---|---|---|---|
| | 0 | ½ | 1 | 2 | 3 |
| 1 | 16.0 | 17.9 | 20.2 | 23.6 | 26.2 |
| 2 | 15.0 | 16.4 | 17.5 | 19.0 | 20.3 |
| 3 | 16.2 | 17.2 | 18.6 | 20.7 | 22.1 |
| 4 | 15.8 | 16.0 | 16.4 | 17.0 | 17.2 |

It is seen from table 2 that addition of copper decreases the formation of heavy chain degradation products. By combining copper with high ionic strength in formulation 4 the lowest increase is obtained.

*Example 4*

Formulation of the following liquid, aqueous pharmaceutical compositions is envisaged:
A)

| | |
|---|---|
| rhFVIIa | 1 mg/mL (approx. 50,000 IU/mL) |
| PIPES | 15.12 mg/mL (50 mM) |
| Copper(II) chloride | 80 $\mu$M |
| Poloxamer 188 | 1.0 mg/mL |
| Sodium chloride | 2.92 mg/mL (50 mM) |
| Calcium chloride 2 $H_2O$ | 1.47 mg/mL (10 mM) |
| Methionine | 0.5 mg/mL |
| 1 M NaOH/1 M HCl | added to pH 6.5 |

B)

| | |
|---|---|
| rhFVIIa | 1 mg/mL (approx. 50,000 IU/mL) |
| PIPES | 15.12 mg/mL (50 mM) |
| Manganese(II) chloride | 10 mM |
| Poloxamer 188 | 1.0 mg/mL |
| Sodium chloride | 2.92 mg/mL (50 mM) |
| Calcium chloride 2 $H_2O$ | 1.47 mg/mL (10 mM) |
| Methionine | 0.5 mg/mL |
| 1 M NaOH/1 M HCl | added to pH 6.5 |

C)

| | |
|---|---|
| rhFVIIa | 1 mg/mL (approx. 50,000 IU/mL) |
| PIPES | 15.12 mg/mL (50 mM) |
| Copper(II) chloride | 80 $\mu$M |
| Poloxamer 188 | 1.0 mg/mL |
| Sodium chloride | 2.92 mg/mL (50 mM) |
| Calcium chloride 2 $H_2O$ | 1.47 mg/mL (10 mM) |
| 1 M NaOH/1 M HCl | added to pH 6.5 |

D)

| | |
|---|---|
| rhFVIIa | 1 mg/mL (approx. 50,000 IU/mL) |
| PIPES | 15.12 mg/mL (50 mM) |
| Manganese(II) chloride | 10 mM |

(continued)

| Poloxamer 188 | 1.0 mg/mL |
|---|---|
| Sodium chloride | 2.92 mg/mL (50 mM) |
| Calcium chloride 2 $H_2O$ | 1.47 mg/mL (10 mM) |
| 1 M NaOH/1 M HCl | added to pH 6.5 |

Pharmaceutical compositions A-D can subsequently be transferred to sterile vials or cartridges, optionally flushed with nitrogen or argon. They may optionally further be packed in air-tight aluminium-laminated plastic bags.

*Example 5*

Addition of copper-containing agents to aqueous rFVIIa solutions with high ionic strength.

In order to investigate the possible synergistic effect of metal ions and high ionic strength on the stability of rFVIIa, the following procedure was followed:

rFVIIa was transferred into various solutions (formulations) as listed in table 1 below in a two step process:

First, rFVIIa was transferred into the various solutions without copper added by desalting on a PD-10 column (Amersham Biosciences). Next, the copper content was obtained in solution 2 and 4 by adding a solution of 10 mM Copper(II) chloride, 2H2O until the stated concentration was reached.

After the copper solution had been added, pH was adjusted to 6.5 and the formulations were filled in cartridges. The cartridges were stored at a temperature of 5°C and analyses were performed after 0, 0.5, 1, 2, and 3 months as indicated in table 2.

Table 1: Formulations

| Formulations | 1 | 2 | 3 | 4 |
|---|---|---|---|---|
| rFVIIa | 1,0 mg/ml | 1,0 mg/ml | 1,0 mg/ml | 1,0 mg/ml |
| CaCl2, 2H2O | 10mM | 10mM | 200mM | 200mM |
| NaCl | 0 mM | 0 mM | 500 mM | 500 mM |
| PIPES-di-Na | 50 mM | 50 mM | 50 mM | 50 mM |
| Cu(II) | - | 80μM | - | 80μM |
| pH | 6.50 | 6.50 | 6.50 | 6.50 |

Table 2: Heavy chain degradation (%)

| Formulation | Storage time (months) | | | | | |
|---|---|---|---|---|---|---|
| | 0 | ½ | 1 | 2 | 3 | Total increase |
| 1 | 11.6 | 15.6 | 19.0 | 24.1 | 27.8 | 16.2 |
| 2 | 11.5 | 13.4 | 15.1 | 18.3 | 20.0 | 8.5 |
| 3 | 12.2 | 13.2 | 15.3 | 16.7 | 18.9 | 6.7 |
| 4 | 12.0 | 12.5 | 13.4 | 14.0 | 15.3 | 3.3 |

It is seen from table 2 that addition of copper to a solution with high ionic strength gives the lowest increase in the formation of heavy chain degradation products.

*Example 6*

Addition of copper-containing agents to aqueous rFVIIa solutions with high ionic strength.

In order to investigate the possible synergistic effect of metal ions and high ionic strength on the stability of rFVIIa, the following procedure was followed:

rFVIIa was transferred into various solutions (formulations) as listed in table 1 below in a two step process:

First, rFVIIa was transferred into the various solutions without copper added by desalting on a PD-10 column (Amersham Biosciences). Next, the copper content was obtained in solution 2 and 4 by adding a solution of 10 mM Copper(II) chloride, 2H2O until the stated concentration was reached.

After the copper solution had been added, pH was adjusted to 6.5 and the formulations were filled in cartridges. The cartridges were stored at a temperature of 5°C and analyses were performed after 0, 3, 7, and 8 months as indicated in table 2.

Table 1: Formulations

| Formulations | 1 | 2 | 3 | 4 |
|---|---|---|---|---|
| rFVIIa | 1,0 mg/ml | 1,0 mg/ml | 1,0 mg/ml | 1,0 mg/ml |
| CaCl2, H2O | 10 mM | 10 mM | 10 mM | 10 mM |
| NaCl | 0 mM | 0 mM | 500mM | 500mM |
| PIPES-di-Na | 50 mM | 50 mM | 50 mM | 50 mM |
| Cu(II) | - | 80μM | - | 80uM |
| pH | 6.50 | 6.50 | 6.50 | 6.50 |

Clotting activity was assayed in a one-stage clot assay essentially as described in Assay 4 of the present specification.

Table 2: Clot activity (%)

| Formulation | Storage time (months) | | | |
|---|---|---|---|---|
| | 0 | 3 | 7 | 8 |
| 1 | 100 | 87 | 68 | 68 |
| 2 | 100 | 91 | 78 | 78 |
| 3 | 100 | 91 | 82 | 83 |
| 4 | 100 | 92 | 89 | 92 |

It is seen from table 2 that addition of copper to a solution with high ionic strength gives the most stable formulation with regard to clot activity.

*Example 7*

Addition of cobalt containing and nickel containing agents to aqueous rFVIIa solutions.
In order to investigate the possible effect of these metal ions on the stability of rFVIIa, the following procedure was followed:

rFVIIa was transferred into various solutions (formulations) as listed in table 1 below in a two step process:

First, rFVIIa was transferred into the various solutions without Co(II) and Ni(II) added by desalting on a PD-10 column (Amersham Biosciences). Next, the Co(II) content was obtained in solution 2 and 3 by adding a solution of 2M Co(II)Cl2 and the content of Ni(II) was obtained in solution 4 and 5 by adding a solution of 2M Ni(II)Cl2 to the stated concentration was reached.

After the Co(II) and Ni(II) solution had been added, pH was adjusted to 6.5 and the formulations were filled in cartridges. The cartridges were stored at a temperature of 5°C and analyses were performed at the times indicated in table 2.

Table 1: Formulations

| Formulations | 1 reference | 2 | 3 | 4 | 5 |
|---|---|---|---|---|---|
| rFVIIa | 1,0 mg/ml | 1,0 mg/ml | 1,0 mg/ml | 1,0 mg/ml | 1,0 mg/ml |
| NaCl | 50 mM | 50 mM | 50 mM | 50 mM | 50 mM |
| CaCl2, 2H2O | 10mM | 10mM | 10mM | 10mM | 10mM |
| PIPES | 50 mM | 50 mM | 50 mM | 50 mM | 50 mM |
| Co(II) | - | 2mM | 4mM | - | - |
| Ni(II) | - | - | - | 6 mM | 12 mM |
| pH | 6.50 | 6.50 | 6.50 | 6.50 | 6.50 |

Table 2: Heavy chain degradation (%)

| Formulation | Storage time (months) | | | | | |
|---|---|---|---|---|---|---|
| | 0 | ½ | 1 | 2 | 3 | Total increase |
| 1 | 11.9 | 14.9 | 16.2 | 22.6 | 26.9 | 12.0 |
| 2 | 11.4 | 12.4 | 12.9 | 15.2 | 16.6 | 5.2 |
| 3 | 11.6 | 12.2 | 12.4 | 13.8 | 15.0 | 3.4 |
| 4 | 11.6 | 11.6 | 11.6 | 12.3 | 12.7 | 1.1 |
| 5 | 11.5 | 11.5 | 11.5 | 11.9 | 12.0 | 0.5 |

It is seen from table 2 that addition of cobalt or nickel to a solution gives the lowest increase in the formation of heavy chain degradation products.

**Claims**

1.  A liquid, aqueous pharmaceutical composition comprising
    a Factor VII polypeptide (i);
    a buffering agent (ii) suitable for keeping pH in the range of from 4.0 to 9.0;
    at least one metal-containing agent (iii), wherein said metal is selected from the group consisting of chromium, manganese, iron, cobalt, nickel, and copper of oxidation state +II; and
    a non-ionic surfactant (iv).

2.  The composition according to any of the preceding claims, wherein the metal-containing agent (iii) is at least one selected from the group consisting of chromium(II) chloride, manganese(II) chloride, iron(II) chloride, cobalt(II) chloride, nickel(II) chloride, and copper(II) chloride.

3.  The composition according to any of the claims 1-2, wherein the metal of the metal-containing agent (iii) is selected from the group consisting of copper and manganese.

4.  The composition according to claim 3, wherein the metal-containing agent (iii) is selected from the group consisting of copper(II) chloride and manganese(II) chloride.

5.  The composition according to any of the preceding claims, wherein the concentration of the metal-containing agent (iii) is at least 1 $\mu$M.

6.  The composition according to any of the preceding claims, wherein the metal of the metal-containing agent (iii) is copper and the concentration of said agent is at least 5 $\mu$M.

7. The composition according to any of the claims 1-5, wherein the metal of the metal-containing agent (iii) is manganese and the concentration of said agent is at least 100 $\mu$M.

8. The composition according to any of the preceding claims, wherein the Factor VII polypeptide is human Factor VIIa.

9. The composition according to any of the preceding claims, wherein the Factor VII polypeptide is a Factor VII sequence variant.

10. The composition according to claim 9, wherein the ratio between the activity of the Factor VII polypeptide and the activity of native human Factor VIIa (wild-type FVIIa) is at least 1.25 when tested In the "In Vitro Proteolysis Assay" as described herein.

11. The composition according to any of the preceding claims, wherein the Factor VII polypeptide is present in a concentration of 0.1-15 mg/mL.

12. The composition according to any of the preceding claims, which has a pH in the range of from about 4.0 to about 8.0.

13. The composition according to any of the preceding claims, wherein the buffering agent (ii) comprises at least one component selected from the group consisting of acids and salts of MES, PIPES, ACES, BES, TES, HEPES, TRIS, glycinamide, phosphoric acid, acetic acid, lactic acid, and succinic acid.

14. The composition according to claim 13, wherein the concentration of the buffering agent (ii) is 1-100 mM.

15. The composition according to any of the preceding claims, wherein the non-ionic surfactant (iv) is at least one selected from the group consisting of polysorbates, poloxamers, polyoxyethylene alkyl ethers, ethylene/polypropylene block co-polymers, polyethyleneglycol (PEG), polyoxyethylene stearates, and polyoxyethylene castor oils.

16. The composition according to any of the preceding claims, further comprising a tonicity modifying agent (v).

17. The composition according to claim 16, wherein the tonicity modifying agent (v) is at least one selected from the group consisting of neutral salts, amino acids, peptides of 2-5 amino acid residues, monosaccharides, disaccharides, polysaccharides, and sugar alcohols.

18. The composition according to claim 17, wherein at least one tonicity modifying agent (v) is a neutral salt selected from the group consisting of sodium salts, potassium salts, calcium salts, and magnesium salts.

19. The composition according to any of claims 17-18, wherein the tonicity modifying agent (v) is sodium chloride in combination with at least one selected from the group consisting of calcium chloride, calcium acetate, magnesium chloride and magnesium acetate.

20. The composition according to any of the preceding claims, wherein the tonicity modifying agent (v) is present in a concentration of at least 1 mM.

21. The composition according to any of the preceding claims, wherein at least one tonicity modifying agent (v) is an ionic strength modifying agent (v/a).

22. The composition according to any of the preceding claims, which has an ionic strength of at least 50.

23. The composition according to any of the preceding claims, which has an osmolality of 300 $\pm$ 50 milliosmol/kg.

24. The composition according to any of the preceding claims, further comprising an antioxidant (vi).

25. The composition according to claim 24, wherein the antioxidant (vi) is selected from L-methionine, D-methionine, methionine analogues, methionine-containing peptides, methionine-homologues, ascorbic acid, cysteine, homocysteine, gluthatione, cystine, and cysstathionine.

26. The composition according to any of claims 24-25, wherein the antioxidant (vi) is present in a concentration of 0.1-5.0 mg/mL.

**27.** The composition according to any of the preceding claims, further comprising a preservative (vii).

**28.** The composition according to claim 27, wherein the preservative (vii) is selected from the group consisting of phenol, benzyl alcohol, orto-cresol, meta-cresol, para-cresol, methyl paraben, propyl paraben, benzalkonium chloride, and benzaethonium chloride.

**29.** The liquid, aqueous pharmaceutical composition according to any of the preceding claims, which comprises:

0.1-15 mg/mL of a Factor VII polypeptide (i);
a buffering agent (ii) suitable for keeping pH in the range of from about 4.0 to about 9.0;
a copper-containing agent (iii) in concentration of at least 5 $\mu$M;
a non-ionic surfactant (iv); and
a tonicity modifying agent (v) in a concentration of at least 5 mM.

**30.** The liquid, aqueous pharmaceutical composition according to any of the claims 1-29, which comprises:

0.1-15 mg/mL of a Factor VII polypeptide (i);
a buffering agent (ii) suitable for keeping pH in the range of from about 4.0 to about 9.0;
a manganese-containing agent (iii) in concentration of at least 100 $\mu$M;
a non-ionic surfactant (iv); and
at least one tonicity modifying agent (v) in a concentration of at least 5 mM.

**31.** The composition according to any of the preceding claims, which is adapted for parenteral administration.

**32.** The composition according to claim 31, which is adapted for subcutaneous, intramuscular or intravenous injection.

**33.** The composition according to any one of claims 1-32, further comprising Calcium ($Ca^{2+}$) and/or Magnesium ($Mg^{2+}$), preferably Calcium, in a concentration of at least about 2 mM.

**34.** A method for preparing a liquid, aqueous pharmaceutical composition of a Factor VII polypeptide, comprising the step of providing the Factor VII polypeptide (i) in a solution comprising
a buffering agent (ii) suitable for keeping pH in the range of from 4.0 to 9.0;
at least one metal-containing agent (iii), wherein said metal is selected from the group consisting of chromium, manganese, iron, cobalt, nickel, and copper of oxidation state +II; and
a non-ionic surfactant (iv).

**35.** A liquid, aqueous pharmaceutical composition as defined in any of the claims 1-33 for use as a medicament.

**36.** Use of a liquid, aqueous pharmaceutical composition as defined in any of the claims 1-33 for the preparation of a medicament for treating a Factor VII-responsive syndrome.

**37.** An air-tight container containing a liquid, aqueous pharmaceutical composition as defined in any of the claims 1-33, and optionally an inert gas.

**38.** The container according to claim 37, wherein the composition does not comprise a preservative (vii).

**39.** A method of lowering the metal ion concentration in a liquid, aqueous pharmaceutical composition, said method comprising the step of contacting a liquid, aqueous pharmaceutical composition according to any of the claims 1-33 with a cation-exchange material.

**Patentansprüche**

**1.** Flüssige, wässrige pharmazeutische Zusammensetzung, umfassend ein Faktor-VII-Polypeptid (i);
einen Puffer (ii), der geeignet ist, den pH-Wert im Bereich von 4,0 bis 9,0 zu halten;
mindestens ein metallhaltiges Mittel (iii), wobei das Metall ausgewählt ist aus der Gruppe, bestehend aus Chrom, Mangan, Eisen, Kobalt, Nickel und Kupfer des Oxidationszustands +II; und
ein nichtionisches Tensid (iv).

**2.** Zusammensetzung nach einem der vorhergehenden Ansprüche, wobei das metallhaltige Mittel (iii) mindestens eines ist, ausgewählt aus der Gruppe, bestehend aus Chrom(II)-chlorid, Mangan(II)-chlorid, Eisen(II)-chlorid, Kobalt(II)-chlorid, Nickel(II)-chlorid und Kupfer(II)-chlorid.

**3.** Zusammensetzung nach einem der Ansprüche 1-2, wobei das Metall des metallhaltigen Mittels (iii) ausgewählt ist aus der Gruppe, bestehend aus Kupfer und Mangan.

**4.** Zusammensetzung nach Anspruch 3, wobei das metallhaltige Mittel (iii) ausgewählt ist aus der Gruppe, bestehend aus Kupfer(II)-chlorid und Mangan(II)-chlorid.

**5.** Zusammensetzung nach einem der vorhergehenden Ansprüche, wobei die Konzentration des metallhaltigen Mittels (iii) mindestens 1 µM beträgt.

**6.** Zusammensetzung nach einem der vorhergehenden Ansprüche, wobei das Metall des metallhaltigen Mittels (iii) Kupfer ist und die Konzentration des Mittels mindestens 5 µM beträgt.

**7.** Zusammensetzung nach einem der Ansprüche 1-5, wobei das Metall des metallhaltigen Mittels (iii) Mangan ist und die Konzentration des Mittels mindestens 100 µM beträgt.

**8.** Zusammensetzung nach einem der vorhergehenden Ansprüche, wobei das Faktor-VII-Polypeptid humaner Faktor VIIa ist.

**9.** Zusammensetzung nach einem der vorhergehenden Ansprüche, wobei das Faktor-VII-Polypeptid eine Faktor-VII-Sequenzvariante ist.

**10.** Zusammensetzung nach Anspruch 9, wobei das Verhältnis der Aktivität des Faktor-VII-Polypeptids zur Aktivität des nativen humanen Faktors VIIa (Wildtyp-FVIIa) beim Test mit dem hier beschriebenen "In-vitro-Proteolyse-Assay" mindestens 1,25 beträgt.

**11.** Zusammensetzung nach einem der vorhergehenden Ansprüche, wobei das Faktor-VII-Polypeptid in einer Konzentration von 0,1-15 mg/ml vorliegt.

**12.** Zusammensetzung nach einem der vorhergehenden Ansprüche, die einen pH-Wert im Bereich von etwa 4,0 bis etwa 8,0 aufweist.

**13.** Zusammensetzung nach einem der vorhergehenden Ansprüche, wobei der Puffer (ii) mindestens einen Bestandteil umfasst, ausgewählt aus der Gruppe, bestehend aus Säuren und Salzen von MES, PIPES, ACES, BES, TES, HEPES, TRIS, Glycinamid, Phosphorsäure, Essigsäure, Milchsäure und Bernsteinsäure.

**14.** Zusammensetzung nach Anspruch 13, wobei die Konzentration des Puffers (ii) 1-100 mM beträgt.

**15.** Zusammensetzung nach einem der vorhergehenden Ansprüche, wobei das nichtionische Tensid (iv) mindestens eines ist, ausgewählt aus der Gruppe, bestehend aus Polysorbaten, Poloxameren, Polyoxyethylenalkylethern, Ethylen/Polypropylen-Blockcopolymeren, Polyethylenglycol (PEG), Polyoxyethylenstearaten und Polyoxyethylenrizinusölen.

**16.** Zusammensetzung nach einem der vorhergehenden Ansprüche, weiterhin umfassend ein die Tonizität modifizierendes Mittel (v).

**17.** Zusammensetzung nach Anspruch 16, wobei das die Tonizität modifizierende Mittel (v) mindestens eines ist, ausgewählt aus der Gruppe, bestehend aus neutralen Salzen, Aminosäuren, Peptiden mit 2-5 Aminosäureresten, Monosacchariden, Disacchariden, Polysacchariden und Zuckeralkoholen.

**18.** Zusammensetzung nach Anspruch 17, wobei mindestens ein die Tonizität modifizierendes Mittel (v) ein neutrales Salz ist, ausgewählt aus der Gruppe, bestehend aus Natriumsalzen, Kaliumsalzen, Calciumsalzen und Magnesiumsalzen.

**19.** Zusammensetzung nach einem der Ansprüche 17-18, wobei das die Tonizität modifizierende Mittel (v) Natriumchlorid in Kombination mit mindestens einem ist, ausgewählt aus der Gruppe, bestehend aus Calciumchlorid, Calciumacetat, Magnesiumchlorid und Magnesiumacetat.

20. Zusammensetzung nach einem der vorhergehenden Ansprüche, wobei das die Tonizität modifizierende Mittel (v) in einer Konzentration von mindestens 1 mM vorliegt.

21. Zusammensetzung nach einem der vorhergehenden Ansprüche, wobei mindestens ein die Tonizität modifizierendes Mittel (v) ein die Ionenstärke modifizierendes Mittel (v/a) ist.

22. Zusammensetzung nach einem der vorhergehenden Ansprüche, die eine Ionenstärke von mindestens 50 aufweist.

23. Zusammensetzung nach einem der vorhergehenden Ansprüche, die eine Osmolalität von $300\pm50$ Milliosmol/kg aufweist.

24. Zusammensetzung nach einem der vorhergehenden Ansprüche, weiterhin umfassend ein Antioxidans (vi).

25. Zusammensetzung nach Anspruch 24, wobei das Antioxidans (vi) ausgewählt ist aus der Gruppe, bestehend aus L-Methionin, D-Methionin, Methioninanalogen, Methionin enthaltenden Peptiden, Methioninhomologen, Ascorbinsäure, Cystein, Homocystein, Gluthation, Cystin und Cystathionin.

26. Zusammensetzung nach einem der Ansprüche 24-25, wobei das Antioxidans (vi) in einer Konzentration von 0,1-5,0 mg/ml vorliegt.

27. Zusammensetzung nach einem der vorhergehenden Ansprüche, weiterhin umfassend ein Konservierungsmittel (vii).

28. Zusammensetzung nach Anspruch 27, wobei das Konservierungsmittel (vii) ausgewählt ist aus der Gruppe, bestehend aus Phenol, Benzylalkohol, ortho-Cresol, meta-Cresol, para-Cresol, Methylparaben, Propylparaben, Benzalkoniumchlorid und Benzethoniumchlorid.

29. Flüssige, wässrige pharmazeutische Zusammensetzung nach einem der vorhergehenden Ansprüche, umfassend:

   0,1-15 mg/ml eines Faktor-VII-Polypeptids (i);
   einen Puffer (ii), der geeignet ist, den pH-Wert im Bereich von etwa 4,0 bis etwa 9,0 zu halten;
   ein kupferhaltiges Mittel (iii) in einer Konzentration von mindestens 5 $\mu$M;
   ein nichtionisches Tensid (iv) und
   ein die Tonizität modifizierendes Mittel (v) in einer Konzentration von mindestens 5 mM.

30. Flüssige, wässrige pharmazeutische Zusammensetzung nach einem der Ansprüche 1-29, umfassend:

   0,1-15 mg/ml eines Faktor-VII-Polypeptids (i);
   einen Puffer (ii), der geeignet ist, den pH-Wert im Bereich von etwa 4,0 bis etwa 9,0 zu halten;
   ein manganhaltiges Mittel (iii) in einer Konzentration von mindestens 100 $\mu$M;
   ein nichtionisches Tensid (iv) und
   mindestens ein die Tonizität modifizierendes Mittel (v) in einer Konzentration von mindestens 5 mM.

31. Zusammensetzung nach einem der vorhergehenden Ansprüche, die für die parenterale Verabreichung ausgebildet ist.

32. Zusammensetzung nach Anspruch 31, die für die subkutane, intramuskuläre oder intravenöse Injektion ausgebildet ist.

33. Zusammensetzung nach einem der Ansprüche 1-32, weiterhin umfassend Calcium (Ca$^{2+}$) und/oder Magnesium (Mg$^{2+}$), vorzugsweise Calcium, in einer Konzentration von mindestens etwa 2 mM.

34. Verfahren zur Herstellung einer flüssigen, wässrigen pharmazeutischen Zusammensetzung eines Faktor-VII-Polypeptids, umfassend den Schritt der Bereitstellung des Faktor-VII-Polypeptids (i) in einer Lösung, umfassend einen Puffer (ii), der geeignet ist, den pH-Wert im Bereich von 4,0 bis 9,0 zu halten;
mindestens ein metallhaltiges Mittel (iii), wobei das Metall ausgewählt ist aus der Gruppe, bestehend aus Chrom, Mangan, Eisen, Kobalt, Nickel und Kupfer des Oxidationszustands +II;
und
ein nichtionisches Tensid (iv).

35. Flüssige, wässrige pharmazeutische Zusammensetzung, wie in einem der Ansprüche 1-33 definiert, zur Anwendung als ein Arzneimittel.

36. Verwendung einer flüssigen, wässrigen pharmazeutischen Zusammensetzung, wie in einem der Ansprüche 1-33 definiert, zur Herstellung eines Arzneimittels zur Behandlung eines Syndroms, das auf Faktor VII reagiert.

37. Luftdichter Behälter, enthaltend eine flüssige, wässrige pharmazeutische Zusammensetzung, wie in einem der Ansprüche 1-33 definiert, und fakultativ ein inertes Gas.

38. Behälter nach Anspruch 37, wobei die Zusammensetzung kein Konservierungsmittel (vii) umfasst.

39. Verfahren zur Senkung der Metallionenkonzentration in einer flüssigen, wässrigen pharmazeutischen Zusammensetzung, wobei das Verfahren den Schritt des Kontaktierens einer flüssigen, wässrigen pharmazeutischen Zusammensetzung nach einem der Ansprüche 1-33 mit einem Kationenaustauschermaterial umfasst.

## Revendications

1. Une composition pharmaceutique aqueuse liquide comprenant un polypeptide de facteur VII (i) ;
un agent tampon (ii) approprié pour maintenir le pH dans la plage de 4,0 à 9,0 ;
au moins un agent contenant du métal (iii), dans lequel ledit métal est choisi dans le groupe consistant en chrome, manganèse, fer, cobalt, nickel et cuivre à l'état d'oxydation +II ; et
un tensioactif non ionique (iv).

2. La composition selon l'une quelconque des revendications précédentes, dans laquelle ledit agent contenant du métal (iii) est au moins un composant choisi dans le groupe consistant en chlorure de chrome (II), chlorure de manganèse (II), chlorure de fer (II), chlorure de cobalt (II), chlorure de nickel (II) et chlorure de cuivre (II).

3. La composition selon l'une quelconque des revendications 1 et 2, dans laquelle le métal de l'agent contenant du métal (iii) est choisi dans le groupe consistant en cuivre et manganèse.

4. La composition selon la revendication 3, dans laquelle l'agent contenant du métal (iii) est choisi dans le groupe consistant en chlorure de cuivre (II) et chlorure de manganèse (II).

5. La composition selon l'une quelconque des revendications précédentes, dans laquelle la concentration de l'agent contenant du métal (iii) est au moins de 1 $\mu$M.

6. La composition selon l'une quelconque des revendications précédentes, dans laquelle le métal de l'agent contenant du métal (iii) est du cuivre et la concentration dudit agent est au moins de 5 $\mu$M.

7. La composition selon l'une quelconque des revendications 1 à 5, dans laquelle le métal de l'agent contenant du métal (iii) est du manganèse et la concentration dudit agent est au moins de 100 $\mu$M.

8. La composition selon l'une quelconque des revendications précédentes, dans laquelle le polypeptide de facteur VII est le facteur VIIa humain.

9. La composition selon l'une quelconque des revendications précédentes, dans laquelle le polypeptide de facteur VII est un variant de séquence du facteur VII.

10. La composition selon la revendication 9, dans laquelle le rapport entre l'activité du polypeptide de facteur VII et l'activité du facteur VIIa humain natif (FVIIa sauvage) est au moins de 1,25 lors d'une évaluation à l'aide du « test de protéolyse *in vitro* » ici décrit.

11. La composition selon l'une quelconque des revendications précédentes, dans laquelle le polypeptide de facteur VII est présent à une concentration de 0,1 à 15 mg/ml.

**12.** La composition selon l'une quelconque des revendications précédentes, qui possède un pH situé dans la plage allant d'environ 4,0 à environ 8,0.

**13.** La composition selon l'une quelconque des revendications précédentes, dans laquelle l'agent tampon (ii) comprend au moins un composant choisi dans le groupe consistant en acides et sels de MES, PIPES, ACES, BES, TES, HEPES, TRIS, glycinamide, acide phosphorique, acide acétique, acide lactique et acide succinique.

**14.** La composition selon la revendication 13, dans laquelle la concentration de l'agent tampon (ii) est de 1 à 100 mM.

**15.** La composition selon l'une quelconque des revendications précédentes, dans laquelle le tensioactif non ionique (iv) est au moins un composant choisi dans le groupe consistant en polysorbates, poloxamères, alkyléther de polyoxyéthylène, copolymères séquencés polypropylène/éthylène, polyéthylèneglycol (PEG), stéarates de polyoxyéthylène et huiles de ricin polyoxyéthylénées.

**16.** La composition selon l'une quelconque des revendications précédentes, comprenant également un agent modifiant la tonicité (v).

**17.** La composition selon la revendication 16, dans laquelle l'agent modifiant la tonicité (v) est au moins un composant choisi dans le groupe consistant en sels neutres, acides aminés, peptides de résidus d'acides aminés 2 à 5, monosaccharides, disaccharides, polysaccharides et polyols.

**18.** La composition selon la revendication 17, dans laquelle au moins un agent modifiant la tonicité (v) est un sel neutre choisi dans le groupe consistant en sels de sodium, sels de potassium, sels de calcium et sels de magnésium.

**19.** La composition selon l'une quelconque des revendications 17 et 18, dans laquelle l'agent modifiant la tonicité (v) est du chlorure de sodium combiné avec au moins un composant choisi dans le groupe consistant en chlorure de calcium, acétate de calcium, chlorure de magnésium et acétate de magnésium.

**20.** La composition selon l'une quelconque des revendications précédentes, dans laquelle l'agent modifiant la tonicité (v) est présent à une concentration d'au moins 1 mM.

**21.** La composition selon l'une quelconque des revendications précédentes, dans laquelle au moins un agent modifiant la tonicité (v) est un agent modifiant la force ionique (v/a).

**22.** La composition selon l'une quelconque des revendications précédentes, qui possède une force ionique d'au moins 50.

**23.** La composition selon l'une quelconque des revendications précédentes, qui possède une osmolalité de $300 \pm 50$ milliosmoles/kg.

**24.** La composition selon l'une quelconque des revendications précédentes, comprenant également un antioxydant (vi).

**25.** La composition selon la revendication 24, dans laquelle l'antioxydant (vi) est choisi parmi L-méthionine, D-méthionine, analogues de la méthionine, peptides contenant de la méthionine, homologues de la méthionine, acide ascorbique, cystéine, homocystéine, gluthation, cystine et cystathionine.

**26.** La composition selon l'une quelconque des revendications 24 et 25, dans laquelle l'antioxydant (vi) est présent à une concentration de 0,1 à 5,0 mg/ml.

**27.** La composition selon l'une quelconque des revendications précédentes, comprenant également un conservateur (vii).

**28.** La composition selon la revendication 27, dans laquelle le conservateur (vii) est choisi dans le groupe consistant en phénol, alcool benzylique, orthocrésol, métacresol, paracrésol, méthylparabène, propylparabène, chlorure de benzalkonium et chlorure de benzéthonium.

**29.** La composition pharmaceutique aqueuse liquide selon l'une quelconque des revendications précédentes, comprenant :

0,1 à 15 mg/ml d'un polypeptide de facteur VII (i) ;
un agent tampon (ii) approprié pour maintenir le pH dans la plage d'environ 4,0 à environ 9,0 ;
un agent contenant du cuivre (iii) à une concentration d'au moins 5 $\mu$M ;
un tensioactif non ionique (iv) ; et
un agent modifiant la tonicité (v) à une concentration d'au moins 5 mM.

**30.** La composition pharmaceutique aqueuse liquide selon l'une quelconque des revendications 1 à 29, comprenant :

0,1 à 15 mg/ml d'un polypeptide de facteur VII (i) ;
un agent tampon (ii) approprié pour maintenir le pH dans la plage d'environ 4,0 à environ 9,0 ;
un agent contenant du manganèse (iii) à une concentration d'au moins 100 $\mu$M ;
un tensioactif non ionique (iv) ; et
au moins un agent modifiant la tonicité (v) à une concentration d'au moins 5 mM.

**31.** La composition selon l'une quelconque des revendications précédentes, qui est adaptée pour l'administration parentérale.

**32.** La composition selon la revendication 31, qui est adaptée pour l'injection sous-cutanée, intramusculaire ou intraveineuse.

**33.** La composition selon l'une quelconque des revendications 1 à 32, comprenant également du calcium ($Ca^{2+}$) et/ou du magnésium ($Mg^{2+}$), de préférence du calcium, à une concentration d'au moins environ 2 mM.

**34.** Une méthode de préparation d'une composition pharmaceutique aqueuse liquide d'un polypeptide de facteur VII, comprenant une étape d'apport du polypeptide de facteur VII (i) dans une solution comprenant
un agent tampon (ii) approprié pour maintenir le pH dans la plage de 4,0 à 9,0 ;
au moins un agent contenant du métal (iii), dans lequel ledit métal est choisi dans le groupe consistant en chrome, manganèse, fer, cobalt, nickel et cuivre à l'état d'oxydation +II ;
et
un tensioactif non ionique (iv).

**35.** Une composition pharmaceutique aqueuse liquide telle que définie dans l'une quelconque des revendications 1 à 33 destinée à être utilisée comme médicament.

**36.** Utilisation d'une composition pharmaceutique aqueuse liquide telle que définie dans l'une quelconque des revendications 1 à 33 pour la préparation d'un médicament destiné à traiter un syndrome réactif au facteur VII.

**37.** Un contenant étanche à l'air contenant une composition pharmaceutique aqueuse liquide telle que définie dans l'une quelconque des revendications 1 à 33, et éventuellement un gaz inerte.

**38.** Le contenant selon la revendication 37, dans lequel la composition ne comprend pas de conservateur (vii).

**39.** Une méthode de réduction de la concentration en ions métalliques dans une composition pharmaceutique aqueuse liquide, ladite méthode comprenant une étape de mise en contact d'une composition pharmaceutique aqueuse liquide selon l'une quelconque des revendications 1 à 33 avec une matière échangeuse de cations.

**REFERENCES CITED IN THE DESCRIPTION**

**Patent documents cited in the description**

- US 2004115590 A
- WO 9422905 A
- US 4784950 A
- US 5997864 A
- WO 9215686 A
- US 5580560 A
- DK 0200189 W
- WO 0238162 A
- WO 9920767 A
- WO 0158935 A
- WO 0183725 A
- WO 0222776 A
- WO 02077218 A
- WO 0327147 A
- WO 0337932 A
- JP 2001061479 B
- EP 0200421 A
- US 4456591 A, Thomas

**Non-patent literature cited in the description**

- **Manning et al.** *Pharmaceutical Research,* 1989, vol. 6, 903-918
- **Cleland et al.** The development of stable protein compositions: A closer look at protein aggregation, deamidation and oxidation. *Critical Reviews in Therapeutic Drug Carrier Systems,* 1993, vol. 10 (4), 307-377
- **Wang et al.** Parenteral compositions of proteins and peptides: Stability and stabilizers. *Journal of Parenteral Science and Technology,* 1988, vol. 42 (2S
- Basic Inorganic Chemistry. John Wiley & Sons, 1987, 53
- **Persson et al.** *J. Biol. Chem.,* 1997, vol. 272, 19919-19924
- **Persson.** *FEBS Letts.,* 1997, vol. 413, 359-363
- **Lino et al.** *Arch. Biochem. Biophys.,* 1998, vol. 352, 182-192
- **Mollerup et al.** *Biotechnol. Bioeng.,* 1995, vol. 48, 501-505
- **Kornfelt et al.** *Arch. Biochem. Biophys.,* 1999, vol. 363, 43-54
- **Wildgoose et al.** *Biochem,* 1990, vol. 29, 3413-3420
- **Solomon.** *Journal of Chemical Education,* 2001, vol. 78 (12), 1691-92
- **James Fritz ; George Schenk.** *Quantitative Analytical Chemistry,* 1979
- **Monroe et al.** *Brit. J. Haematol.,* 1997, vol. 99, 542-547
- **Hagen et al.** *Proc.Natl.Acad.Sci. USA,* 1986, vol. 83, 2412-2416
- **Broze ; Majerus.** *J.Biol.Chem.,* 1980, vol. 255 (4), 1242-1247
- **Hedner ; Kisiel.** *J.Clin.Invest.,* 1983, vol. 71, 1836-1841
- **Bjoern et al.** *Research Disclosure,* September 1986, vol. 269, 564-565
- **Cunningham ; Wells.** *Science,* 1989, vol. 244, 1081-1085
- **de Vos et al.** *Science,* 1992, vol. 255, 306-312
- **Smith et al.** *Journal of Molecular Biology,* 1992, vol. 224, 899-904
- **Wlodaver et al.** *FEBS Letters,* 1992, vol. 309, 59-64
- **Wakabayashi et al.** *J. Biol. Chem.,* 1986, vol. 261, 11097
- **Thim et al.** *Biochem.,* 1988, vol. 27, 7785
- **Scopes.** Protein Purification. Springer-Verlag, 1982
- Protein Purification. VCH Publishers, 1989
- **Osterud et al.** *Biochem.,* 1972, vol. 11, 2853
- **Hedner et al.** *J. Clin. Invest.,* 1983, vol. 71, 1836